# EUROPÄISCHE PATENTSCHRIFT

(11) **EP 2 580 574 B1**
(45) Veröffentlichungstag und Bekanntmachung des Hinweises auf die Patenterteilung: **23.09.2015**
(21) Anmeldenummer: 11724970.6
(22) Anmeldetag: 09.06.2011
(51) Int. Cl.: G01N 21/65, G01N 15/14, G01N 21/45, G03H 1/04

(54) **VERFAHREN UND VORRICHTUNG ZUM CHARAKTERISIEREN VON BIOLOGISCHEN OBJEKTEN**
METHOD AND APPARATUS FOR CHARACTERIZING BIOLOGICAL OBJECTS
PROCÉDÉ ET DISPOSITIF POUR CARACTÉRISER DES OBJETS BIOLOGIQUES

(30) Priorität: 09.06.2010 DE 102010023099
(43) Veröffentlichungstag der Anmeldung: 17.04.2013
(73) Patentinhaber: CellTool GmbH, 82327 Tutzing (DE)
(72) Erfinder: SCHÜTZE, Raimund, 82327 Tutzing (DE); SCHÜTZE, Karin, 82327 Tutzing (DE)
(74) Vertreter: Meier, Florian
(86) Internationale Anmeldenummer: PCT/EP2011/002839
(87) Internationale Veröffentlichungsnummer: WO 2011/154143

(56) Entgegenhaltungen:
- DE-A1- 10 352 416
- US-A1- 2004 012 778
- F. Schaal ET AL: "Marker-free cell discrimination by holographic optical tweezers", Journal of the European Optical Society - Rapid Publications, 3. Juni 2009 (2009-06-03), Seiten 09028-1, XP55006233, DOI: 10.2971/jeos.2009.09028] Gefunden im Internet: URL:http://www.jeos.org/index.php/jeos_rp/ article/viewFile/09028/426 [gefunden am 2011-09-05]
- BJÖRN KEMPER ET AL: "Monitoring of laser micromanipulated optically trapped cells by digital holographic microscopy", JOURNAL OF BIOPHOTONICS, Bd. 3, Nr. 7, 8. Juni 2010 (2010-06-08), Seiten 425-431, XP55006175, ISSN: 1864-063X, DOI: 10.1002/jbio.201000035
- SATISH RAO ET AL: "Polarization Raman study of protein ordering by controllable RBC deformation", JOURNAL OF RAMAN SPECTROSCOPY, Bd. 40, Nr. 9, 1. September 2009 (2009-09-01), Seiten 1257-1261, XP55006226, ISSN: 0377-0486, DOI: 10.1002/jrs.2275
- S RAO ET AL: "Raman Study of Mechanically Induced Oxygenation State Transition of Red Blood Cells Using Optical Tweezers", BIOPHYSICAL JOURNAL, Bd. 96, Nr. 1, 7. Januar 2009 (2009-01-07) , Seiten 209-216, XP55010712, ISSN: 0006-3495, DOI: 10.1529/biophysj.108.139097
- MEHDI DANESHPANAH ET AL: "3D Holographic Imaging and Trapping for Non-Invasive Cell Identification and Tracking", JOURNAL OF DISPLAY TECHNOLOGY, IEEE SERVICE CENTER, NEW YORK, NY, US, Bd. 6, Nr. 10, 1. Oktober 2010 (2010-10-01), Seiten 490-499, XP011308944, ISSN: 1551-319X
- NELSON CARDENAS ET AL: "Probing orientation and rotation of red blood cells in optical tweezers by digital holographic microscopy", PROCEEDINGS OF SPIE, 1. Januar 2011 (2011-01-01), Seiten 790613-790613-9, XP55006235, ISSN: 0277-786X, DOI: 10.1117/12.876001
- NELSON CARDENAS ET AL: "Stretching of red blood cells by optical tweezers quantified by digital holographic microscopy", PROCEEDINGS OF SPIE, 1. Januar 2011 (2011-01-01), Seiten 78971J-78971J-9, XP55006234, ISSN: 0277-786X, DOI: 10.1117/12.875981
- KEMPER B ET AL: "Modular digital holographic microscopy system for marker free quantitative phase contrast imaging of living cells", PROCEEDINGS OF SPIE, THE INTERNATIONAL SOCIETY FOR OPTICAL ENGINEERING SPIE, USA, Bd. 6191, 1. Januar 2006 (2006-01-01), Seiten 61910T-1, XP003027053, ISSN: 0277-786X, DOI: 10.1117/12.662781
- GAJENDRA P. SINGH ET AL: "Real-Time Detection of Hyperosmotic Stress Response in Optically Trapped Single Yeast Cells Using Raman Microspectroscopy", ANALYTICAL CHEMISTRY, Bd. 77, Nr. 8, 1. April 2005 (2005-04-01), Seiten 2564-2568, XP55010691, ISSN: 0003-2700, DOI: 10.1021/ac048359j
- K SVOBODA ET AL: "Biological Applications of Optical Forces", ANNUAL REVIEW OF BIOPHYSICS AND BIOMOLECULAR STRUCTURE, Bd. 23, Nr. 1, 1. Juni 1994 (1994-06-01), Seiten 247-285, XP55010743, ISSN: 1056-8700, DOI: 10.1146/annurev.bb.23.060194.001335

## Beschreibung

Die Erfindung betrifft ein Verfahren und eine Vorrichtung zum Charakterisieren von biologischen Objekten, z.B. einzelnen Zellen. Die Erfindung betrifft insbesondere ein Verfahren und eine Vorrichtung, mit denen biologische Objekte in einem Mikrofluidiksystem berührungslos charakterisiert und dann sortiert werden können.

Verschiedene Methoden zur Beobachtung und Manipulation biologischer Objekte sind bekannt, mit denen die gezielte Manipulation und Beobachtung für immer kleinere Objekte, beispielsweise einzelne Zellen, möglich wurde. Verschiedene Methoden zur Analyse biologischer Objekte setzen eine gezielte Stimulierung der biologischen Objekte ein. Zu derartigen Methoden gehört beispielsweise die Manipulation von einzelnen Zellen zur Messung ihrer Elastizitätseigenschaften unter Verwendung eines Rasterkraftmikroskops, wie sie in M. Radmacher et al., "Measuring the Viscoelastic Properties of Human Platelets with the Atomic Force Microscope", Biophys. J. 70, 556 (1996) beschrieben ist. Traditionell werden derartige Methoden häufig eingesetzt, um Kenntnisse über den Aufbau, die Funktionsweise und die Eigenschaften von biologischen Objekten zu gewinnen.

Die DE 10 2005 036 326 A1 und die WO 2007/014622 A1 beschreiben ein Verfahren und eine Vorrichtung zur Analyse von biologischen Objekten. Dabei wird eine Form- oder Volumenänderung eines biologischen Objekts anhand von digitaler holographischer Mikrointerferenz (DHMI) beobachtet. Derartige Messungen liefern jedoch keine Informationen über Veränderungen im Inneren des biologischen Objekts.

Um Eigenschaften im Zellinneren zu ermitteln, kann Raman-Spektroskopie eingesetzt werden. Zur Zellcharakterisierung und -Sortierung kann verwendet werden, dass sich beispielsweise das Raman-Spektrum lebender Zellen vom Raman-Spektrum toter Zellen unterscheidet. Anhand des Raman-Spektrums kann somit ermittelt werden, ob eine Zelle tot ist oder lebt. Methoden, bei denen Raman-Spektroskopie an biologischen Objekten durchgeführt wird, sind herkömmlich darauf beschränkt, dass für ein biologisches Objekt nur genau ein Raman-Spektrum erfasst wird. Dies kann eine Charakterisierung beispielsweise in Fällen erschweren, in denen unterschiedliche biologische Objekte unter den vorliegenden Messbedingungen ähnliche oder identische Raman-Spektren aufweisen.

Die schonende, berührungslose und möglichst markerfreie Charakterisierung biologischer Objekte gewinnt zunehmend Bedeutung, beispielsweise bei der biologischen Forschung, der Stammzellforschung, in der genetischen Forschung, der medizinischen Diagnose oder in forensischen Wissenschaften. Bei derartigen Anwendungen ist Zeit ein wichtiger Faktor. Für praktische Applikationen ist es wichtig, einen ausreichenden Durchsatz von biologischen Objekten bei der Charakterisierung zu erreichen. Falls biologische Objekte einzeln mechanisch stimuliert werden, kann es schwierig sein, einen ausreichenden Durchsatz zu erreichen. Das Setzen eines Stimulus kann auch unter Verwendung von biochemischen Substanzen erfolgen. Dies kann jedoch dazu führen, dass abhängig von dem jeweils interessierenden biologischen Objekt und der verwendeten Substanz das biologische Objekt für eine weitere Analyse unbrauchbar wird.

S. Rao et al., "Polarization Raman study of protein ordering by controllable RBC deformation", J. Raman Spectrosc., 2009, 40, 1257-1261, offenbart die Messung des Raman-Spektrums roter Blutkörperchen vor und nach einer Ausübung mechanischer Spannungen auf dieselben durch optische Pinzetten, um die Abhängigkeit der Ausrichtung von Hämoglobin von der Zelldeformation zu untersuchen. Eine ähnliche Messung offenbart S. Rao et al., "Raman study of mechanically induced oxygenation state transition of red blood cells using optical tweezers", Biophys. J., 2009, 96, 209-216, zur Untersuchung der Abhängigkeit des Oxygenierungszustands der roten Blutkörperchen von Verspannungen der Zelle, die durch eine optischen Pinzette verursacht werden.

Für praktische Anwendungen besteht darüber hinaus ein Bedarf daran, die Charakterisierung biologischer Objekte noch genauer durchzuführen. Zudem ist eine weitgehende Automatisierung erwünscht. Eine automatische Erkennung der gewünschten biologischen Objekte ist mit herkömmlichen Verfahren und Vorrichtungen häufig nicht oder nur mit großen Aufwand möglich ist. Dies liegt teilweise daran, dass bei herkömmlichen Verfahren und Vorrichtungen typischerweise nur eine Methode zur Beobachtung eingesetzt wird oder beispielsweise dreidimensionale Objekte nur zweidimensional dargestellt werden. Eine dreidimensionale Darstellung erfordert bei Verwendung herkömmlicher Methoden, beispielsweise eines Laser-Scannens, ein zeitintensives Scannen verschiedener Ebenen und eine anschließende digitale Zusammenführung der Bilddaten. Dies erschwert die weitergehende Automatisierung der Charakterisierung von Zellen.

Der Erfindung liegt die Aufgabe zugrunde, ein Verfahren und eine Vorrichtung zur Charakterisierung von biologischen Objekten anzugeben, wobei eine einfache, schnelle und genaue Charakterisierung von einzelnen biologischen Objekten möglich ist. Insbesondere liegt der Erfindung die Aufgabe zugrunde, ein derartiges Verfahren und eine derartige Vorrichtung anzugeben, bei der ein Stimulus auf das biologische Objekt ausgeübt wird und die nicht ausschließlich auf der Verwendung zweidimensionaler Darstellungen einer äußeren Veränderung des biologischen Objekts beruht, sondern Informationen über Vorgänge im Zellinneren liefert.

Diese Aufgabe wird erfindungsgemäß durch ein Verfahren und eine Vorrichtung gelöst, wie sie in den unabhängigen Ansprüchen angegeben sind. Die abhängigen Ansprüche definieren Ausführungsformen der Erfindung.

Erfindungsgemäß wird zur Charakterisierung von biologischen Objekten ein Reiz (Stimulus) auf ein biologisches Objekt ausgeübt und eine Reaktion des biologischen Objekts unter Verwendung von Raman-Streuung und vorzugsweise zusätzlich einer digitalen holographischen Mikrointerferometrie (DHMI) gemessen. Die DHMI wird in der Technik auch als digital-holographische Mikroskopie bezeichnet.

Zum Ermitteln der Reaktion des biologischen Objekts wird
- eine Erfassung der Raman-Streuung vor Ausüben des Reizes durchgeführt und vorzugsweise zusätzlich eine DHMI-Messung zur Brechungsindexbestimmung, sowie
- eine weitere Erfassung der Raman-Streuung sowie vorzugsweise zusätzlich eine DHMI-Messung zur Brechungsindexbestimmung nach oder während Ausüben des Reizes durchgeführt, wobei das biologische Objekt abhängig von einem Vergleich der Resultate der Messungen charakterisiert wird.

Bei dem Verfahren und der Vorrichtung kann insbesondere die intrinsische Reaktion auf den Reiz beobachtet werden. Vorteilhaft kann die Reaktion auf den Reiz sowohl im Hinblick auf extrinsische Parameter, beispielsweise die Form oder das Volumen des Objekts, als auch im Hinblick auf intrinsische Parameter, beispielsweise die lokale Molekülzusammensetzung oder den lokalen Brechungsindex in der Zelle, ermittelt werden. Das Verfahren und die Vorrichtung erlauben eine umfassendere Charakterisierung von Zellen. Beispielsweise können äußere Veränderungen nicht nur zweidimensional sondern dreidimensional dargestellt werden. Mittels der DHMI und Ramanspektroskopie können die intrinsischen Reaktionen auf den Reiz analysiert werden.

Die Charakterisierung des biologischen Objekts erfolgt anhand des Vergleichs von Messresultaten, die vor Reizsetzung und während oder nach Reizsetzung ermittelt werden. Dadurch wird größere Flexibilität bei der Charakterisierung biologischer Objekte erreicht. Selbst wenn unterschiedliche biologische Objekte beispielsweise unter gegebenen Messbedingungen ähnliche Raman-Spektren aufweisen, kann mit dem Verfahren und der Vorrichtung nach Ausführungsbeispielen eine Veränderung im Raman-Spektrum erfasst werden. Dies erlaubt die Unterscheidung von Objekten, die zwar unter bestimmten Messbedingungen ähnliche Raman-Spektren aufweisen, aber deren Raman-Spektren als Reaktion auf die Reizausübung unterschiedliche Veränderungen aufweist. Beispielsweise kann während der Reizsausübung eine das biologische Objekt charakterisierende Verschiebung eines Raman-Spektrums, einzelner Spektrallinien oder spektraler Gewichte vorliegen. Unterschiedliche biologische Objekte können auch unterschiedliche Rückkehrverhalten nach Reizausübung aufweisen. Diese können als Funktion der Zeit im Raman-Spektrum beobachtet und zur Charakterisierung verwendet werden.

Entsprechend kann unter Verwendung von DHMI zur Brechungsindexbestimmung die Reaktion des biologischen Objekts auf einen Reiz beobachtet werden. Dabei kann der Brechungsindex zu mehreren Zeiten erfasst werden, um ein Rückkehrverhalten des biologischen Objekts nach Reizsetzung zu beobachten, das zur Charakterisierung eingesetzt werden kann.

Biologische Objekte können selbst dann, wenn sie eine ähnliche Morphologie oder Topologie aufweisen und somit durch herkömmliche 2D-Abbildungen nicht einfach unterschieden werden können, signifikant unterschiedliches Reaktionsverhalten nach Ausüben eines Reizes zeigen. Mit der Raman-Streuung kann das Streuverhalten des biologischen Objekts erfasst werden. Anhand der Frequenzverschiebungen des an dem biologischen Objekt gestreuten Lichts kann ein charakteristisches Verhalten des Objekts, beispielsweise im Hinblick auf Schwingungsspektren von Molekülen und somit die vorhandenen Moleküle, erfasst werden. Da bei der Messung der Raman-Streuung mehrere Spektrallinien eines Schwingungsspektrums oder mehrerer Schwingungsspektren erfasst werden können, kann ein charakteristisches Schwingungsspektrum nach Ausüben des Reizes auf das biologische Objekt erfasst werden. Beispielsweise lassen sich einzelne Moleküle identifizieren. Durch die Erfassung des Raman-Spektrums können chemische Veränderungen dargestellt werden, die beispielsweise krankhaften Zellen bei Tumorgeweben zugeschrieben werden. Auf diese Weise können beispielsweise Tumorzellen von gesunden Zellen unterschieden werden. Raman-Spektren können aber auch Aufschluss über das Differenzierungsstadium von Zellen geben und somit Stammzellen von Körperzellen unterscheiden.

Die digitale holographische Mikrointerferometrie (DHMI) ist ein holographisches Messverfahren. Zwei phasenkohärente Teilstrahlen, ein Objektstrahl und ein Referenzstrahl, werden überlagert. Das resultierende Interferenzbild wird von einem elektronischen Bildsensor, beispielsweise einem CCD-Sensor, erfasst. Aus einer einzigen DHMI-Aufnahme können verschiedene Objektebenen rekonstruiert werden, so dass zusätzliche Information zur Charakterisierung von Zellen zur Verfügung steht. Mit der DHMI lassen sich indirekt molekulare Veränderungen innerhalb der einzelnen Zellen darstellen, so dass sich die Zelltypen gemäß ihrer morphologischen Eigenschaften wie Schichtdicke, Volumen, Mikrobewegungen oder Verformungen schnell und berührungsfrei charakterisieren lassen. Aufgrund dieser Eigenschaften können mit der DHMI-Datenerfassung auch dynamische Prozesse nach Ausüben eines Reizes auf das biologische Objekt beobachtet und zur Charakterisierung des biologischen Objekts herangezogen werden. Eine zuverlässige Analyse des biologischen Objekts ist auch bei optischer Unschärfe möglich, beispielsweise bei Bewegung des Objekts in Tiefenrichtung während der Datenerfassung, da eine Fokuskorrektur automatisch und rechnergestützt durchgeführt werden kann. Eine mechanische Fokuskorrektur kann entfallen. Dies ermöglicht eine sehr schnelle Datenerfassung ohne langwieriges Scannen und dient somit der Steigerung des Durchsatzes.

Bei einer Ausführungsform kann eine DHMI-Datenerfassung an einer Zelle durchgeführt werden, um ein Verhältnis von Kern zu Zytoplasma zu ermitteln. Alternativ oder zusätzlich können typische Kern-Muster anhand ihres unterschiedlichen Brechungsverhaltens quantifiziert werden. So kann das Verfahren und die Vorrichtung nach Ausführungsformen der Erfindung zur Zytometrie eingesetzt werden.

Bei den Verfahren und Vorrichtungen wird der Reiz vorteilhaft so ausgeübt, dass das biologische Objekt nicht zerstört oder geschädigt wird. Die auf das biologische Objekt eingestrahlte Leistung für die Raman-Spektroskopie und vorzugsweise zusätzlich die DHMI wird so gewählt, dass sie ebenfalls nicht zu einer Zerstörung des biologischen Objekts führt. Somit bleibt das biologische Objekt sowohl vor als auch nach Reizsetzung ein intaktes biologisches Objekt. Mit der Raman-Spektroskopie und vorzugsweise zusätzlich mit der DHMI lassen sich charakteristische Eigenschaften biologischer Objekte ermitteln, ohne dass dafür eine Markierung des biologischen Objekts erforderlich wäre.

Mit dem erfindungsgemäßen Verfahren und der erfindungsgemäßen Vorrichtung ist somit eine berührungsfreie und markerfreie Charakterisierung eines biologischen Objekts möglich, die das biologische Objekt nicht zerstört. Biologische Objekte können dabei anhand ihrer Reaktion auf einen Reiz charakterisiert werden, die durch Raman-Spektroskopie und vorzugsweise zusätzlich DHMI gemessen wird.

Die weitere Raman-Spektroskopie und vorzugsweise zusätzlich weitere Datenerfassung für die DHMI zur Brechungsindexbestimmung erfolgt, nachdem der Reiz ausgeübt wurde oder während der Reiz ausgeübt wird. Auf diese Weise kann beispielsweise das Rückkehrverhalten eines biologischen Objekts, beispielsweise einer Zelle, nach einer durch den Reiz verursachen Veränderung beobachtet werden. Es können zeitsequentiell mehr als zwei Raman-Spektren erfasst werden, um die Reaktion des biologischen Objekts als Funktion der Zeit auf den Reiz zu beobachten.

Es kann rechnerisch ein Differenzspektrum eines Raman-Spektrums, das in einer Messung erfasst wurde, und eines weiteren Raman-Spektrums, das in einer weiteren Messung erfasst wurde, ermittelt werden. Das Differenzspektrum kann mit Referenz-Differenzspektren abgeglichen werden, die in einer Datenbank abgelegt sind, um das biologische Objekt zu charakterisieren.

Eine für die Raman-Spektroskopie verwendete Laserlichtquelle kann auch zur Erzeugung einer Resonanzschwingung des biologischen Objekts verwendet werden. Dabei wird zunächst ein von der Laserlichtquelle ausgegebenes Licht gepulst mit einer Repetitionsrate auf das biologische Objekt eingestrahlt. Die Repetitionsrate kann variiert werden, um eine Resonanzschwingung des biologischen Objekts anzuregen. Das Erreichen der Resonanz kann durch Beobachtung der 3D-Form überwacht werden. Alternativ oder zusätzlich kann das Erreichen der Resonanz auch anhand eines erfassten Raman-Spektrums überwacht werden. Bei Erreichen der Resonanz kann beispielsweise die Laserintensität reduziert werden, um eine Zerstörung des biologischen Objekts zu vermeiden. Während das biologische Objekt Resonanzschwingungen ausführt, können Veränderungen im Inneren des biologischen Objekts anhand von Raman-Spektroskopie und vorzugsweise zusätzlich DHMI zur Brechungsindexbestimmung beobachtet werden.

Die an dem biologischen Objekt durchgeführte Messung umfasst eine Raman-Spektroskopie und vorzugsweise zusätzlich eine Datenerfassung mittels DHMI.

Durch die Kombination von Raman-Spektroskopie und DHMI zur Formmessung kann sowohl eine äußere Veränderung des biologischen Objekts, beispielsweise eine Veränderung von Form oder Volumen, als auch eine Veränderung im Inneren des biologischen Objekts ermittelt werden. Die ermittelte Veränderung im Inneren, die auch als intrinsische Veränderung bezeichnet wird, kann eine Veränderung des Brechungsindex, die durch DHMI beobachtet wird, oder eine Veränderung des Streuungsverhaltens im Raman-Spektrum als Reaktion auf den Reiz sein. Im Vergleich zu Methoden, die nur Änderungen der Form des Objekts erfassen, können somit zusätzliche Informationen gewonnen werden, die zur Charakterisierung des Objekts eingesetzt werden können. Mit DHMI lassen sich zusätzlich Form- und Volumenänderungen nach Ausüben eines Reizes auf das biologische Objekt ermitteln. Bei zusätzlicher Durchführung einer Raman-Spektroskopie können auch Aussagen zur Veränderungen des erfassten Raman-Spektrums und somit des Schwingungsspektrums gewonnen. Beispielsweise kann eine Änderung der Protein- oder Molekülzusammensetzung ermittelt werden. Eine Korrelation der mit DHMI und Raman-Spektroskopie gewonnen Ergebnisse kann durchgeführt werden, um das biologische Objekt genauer charakterisieren oder identifizieren zu können.

Der Reiz kann markerfrei ausgeübt werden. Auf diese Weise kann sichergestellt werden, dass das biologische Objekt auch nach seiner Charakterisierung für weitere Untersuchungen zur Verfügung steht. Dies ist insbesondere dann vorteilhaft, wenn die Charakterisierung für eine Sortierung lebender Zellen erfolgt.

Der Reiz kann unter Verwendung optischer Strahlung auf das biologische Objekt ausgeübt werden. Der Reiz kann so ausgeübt werden, dass eine Verformung des biologischen Objekts hervorgerufen wird. Hierzu können verschiedene Methoden eingesetzt werden. Nach einer Ausführungsform kann wenigstens eine optische Pinzette oder Falle unter Verwendung eines oder mehrerer Laserstrahlen erzeugt werden. Bei einer Ausführungsform wird unter Verwendung wenigstens eines Laserstrahls eine optische Pinzette erzeugt, mit der das biologische Objekt in einem Fluidstrom mit endlicher Strömungsgeschwindigkeit gehalten wird. Dabei kann die Laserleistung und somit die Tiefe des Fallenpotenzials sowie die Strömungsgeschwindigkeit des Fluidstroms so eingestellt werden, dass die von dem Fluid auf das biologische Objekt ausgeübten Scherkräfte zu einer Verformung des biologischen Objekts führen. So wird eine Verformung des biologischen Objekts durch das Zusammenwirken von Fluidkräften und optischen Kräften erreicht. Der Laserstrahl kann entlang einem Fluidkanal und entgegen dem Fluidstrom gerichtet sein. Dabei können in Längsrichtung des Fluidkanals mehrere biologische Objekte gleichzeitig gehalten werden.

Bei Ausführungsformen kann die optische Pinzette, mit der das Objekt an einer gewünschten Stelle gehalten wird, durch den zur Durchführung der Raman-Spektroskopie auf das Objekt eingestrahlten Strahl gebildet werden.

Bei einer weiteren Ausführungsform kann zum Ausüben des Reizes ein Paar von optischen Pinzetten verwendet werden, mit denen an entgegengesetzten Seiten des biologischen Objekts Kräfte erzeugt werden können, so dass beispielsweise eine Verformung des Objekts resultiert. Dabei können die beiden optischen Pinzetten lateral bewegt werden, um beispielsweise eine Streckung des Objekts hervorzurufen. Bei einer weiteren Ausführungsform kann ein Laserstrahl oder ein Paar von im Wesentlichen gegenläufigen Laserstrahlen verwendet werden, deren Leistung und Profil so gewählt wird, dass Kräfte auf das biologische Objekt ausgeübt werden, die das biologische Objekt in Propagationsrichtung der Laserstrahlen zusammendrücken oder strecken. Bei einer weiteren Ausführungsform kann mit einem Mikrostrahl oder zwei gegenläufigen Mikrostrahlen die Zelle deformiert werden. Als Mikrostrahl wird hierbei vorteilhaft ein Laserstrahl eingesetzt, mit dem nur kurzzeitig Licht auf das biologische Objekt eingestrahlt wird. Beispielsweise kann ein gepulster Laser verwendet werden, um den Mikrostrahl zu erzeugen. Alternativ kann, beispielsweise unter Verwendung eines elektrooptischen Elements, der Strahl eines kontinuierlich arbeitenden Laser so beeinflusst werden, dass er für eine kurze Zeitspanne als Mikrostrahl auf das biologische Objekt trifft. Mit einem Mikrostrahl kann auch eine lokale Störung auf der Membran und/oder im Zytoplasma einer Zelle hervorgerufen werden, z.B. durch Optoperforation bzw. Optoporation. Die resultierende Änderung der Proteinzusammensetzung kann mit Raman-Spektroskopie detektiert und/oder die Änderung der Morphologie bzw. des Brechungsindex der Zelle mit DHMI erfasst werden.

Bei weiteren Ausführungsformen kann mit optischen Methoden auch ohne induzierte Verformung eines biologischen Objekts ein Reiz gesetzt werden, der beispielsweise eine lokale Erwärmung umfasst. Die Reaktion wird mit Raman-Spektroskopie und vorzugsweise zusätzlich DHMI erfasst.

Bei jeder dieser Ausführungsformen kann der Strahlengang des Laserstrahls bzw. der Laserstrahlen über verschiedene Positionen gerastert werden. Auf diese Weise können Reize auf biologische Objekte, die an unterschiedlichen Messpositionen gehalten werden, ausgeübt werden. Es kann eine Ablenkeinrichtung vorgesehen sein, die den Laserstrahl bzw. die Laserstrahlen in kontrollierbarer Weise rastert. Bei einer Ausführungsform kann dazu ein optisches Element, z.B. ein Spiegel, kontrolliert verstellt werden. Bei einer weiteren Ausführungsform kann ein Laserstrahl auch mittels eines SLM (räumlicher Lichtmodulator)-Kristalls über verschiedene Positionen gerastert werden. Bei einer Ausführungsform kann die Rasterung so erfolgen, dass der Laserstrahl bzw. die Laserstrahlen zeitsequentiell auf verschiedene Fluidkanäle gerichtet wird bzw. werden. Alternativ oder zusätzlich kann die Rasterung so erfolgen, dass der Laserstrahl bzw. die Laserstrahlen auf verschiedene in Längsrichtung oder Transversalrichtung eines Kanals beabstandete Messpositionen gerichtet wird, um den Reiz auf verschiedene biologische Objekte auszuüben. Bei einer Ausführungsform erfolgt eine Rasterung in zwei Dimensionen, bei der der Laserstrahl bzw. die Laserstrahlen sowohl zwischen verschiedenen Fluidkanälen als auch entlang der Längsrichtung der Kanäle zwischen verschiedenen Positionen verstellt werden, an denen ein Reiz auf biologische Objekte ausgeübt werden soll. Falls zum Ausüben des Reizes mindestens zwei Laserstrahlen auf ein biologisches Objekt gerichtet werden, werden die zwei Laserstrahlen synchron gerastert. Durch die genannten Maßnahmen kann eine Parallelisierung bzw. ein Multiplexing bei der Charakterisierung der biologischen Objekte erfolgen, die eine weitere Erhöhung des Durchsatzes ermöglicht.

Es kann eine Datenerfassung mittels DHMI vorgenommen werden, nachdem unter Verwendung optischer Methoden eine Verformung des biologischen Objekts hervorgerufen wurde. Zusätzlich erfolgt eine Datenerfassung vor Setzen des Reizes, um die Reaktion des biologischen Objekts beurteilen zu können. Dadurch können Zellen oder andere biologische Objekte und ihre Veränderung in drei Dimensionen rekonstruiert und die Reaktion auf den Reiz quantifiziert werden. Aus einer einzelnen DHMI-Aufnahme können verschiedene Schnitte des biologischen Objekts rekonstruiert werden. Bei einer Verformung als Reaktion auf einen optisch induzierten Reiz resultiert im Allgemeinen eine Änderung nicht nur eines Querschnitts des biologischen Objekts, sondern typischerweise eine Änderung in drei Dimensionen. Für ein nicht homogenes Objekt, wie beispielsweise eine Zelle, ist die Reaktion auf Druck- oder Zugkräfte in den drei Dimensionen unterschiedlich stark. Mit der 3D-Bildgebung mittels DHMI erhält man genauere Aussagen über das Elastizitätsverhalten in den unterschiedlichen Richtungen. Eine digitale Nachfokussierung bei der rechnergestützten Auswertung der DHMI-Daten erlaubt es, auf eine mechanische Fokuskorrektur zu verzichten. Bei der DHMI kann darüber hinaus mit einer einzigen Momentaufnahme das gesamte Zellvolumen erfasst werden, so dass auf ein Scannen verzichtet werden kann. Auf diese Weise kann der Durchsatz bei der Charakterisierung erhöht werden.

Durch eine DHMI-Datenerfassung wird der Brechungsindex einer Zelle bzw. von Komponenten der Zelle, wie dem Zellkern, quantitativ bestimmt. Zur Bestimmung des Brechungsindex können mehrere DHMI-Datenerfassungen vorgenommen werden, wobei die Zelle zwischen den verschiedenen DHMI-Datenerfassungen gedreht oder in ihrer Lage verändert wird. Eine Drehung der Zelle kann beispielsweise durch den Laserstrahl bzw. die Laserstrahlen hervorgerufen werden, die auch zum Ausüben des Reizes auf die Zelle eingesetzt werden. Ein Laserstrahlprofil, das nicht rotationssymmetrisch ist, kann verwendet werden, um eine Rotation der Zelle hervorzurufen. Dieses kann beispielsweise erzeugt werden, indem eine optische Faser mit elliptischem Querschnitt verwendet wird. Es können auch Laserstrahlen verwendet werden, die eine helische Wellenfront aufweisen. Beispielsweise können gegenläufige Laserstrahlen eingesetzt werden, die ein Laguerre-Gauss-Profil und eine helische Wellenfront aufweisen, so dass der Poynting-Vektor sprialförmig entlang der die Propagationsrichtung definierenden Achse variiert. Derartige Strahlen übertragen ein Drehmoment auf das biologische Objekt, mit dem dieses gedreht werden kann. Alternativ oder zusätzlich können optische Fasern eingesetzt werden, die Strahlung bereitstellen, mit der das biologische Objekt gedreht werden kann. Die optischen Fasern können einen nicht rotationssymmetrischen Querschnitt aufweisen. Beispielsweise können die Fasern einen elliptischen Querschnitt aufweisen.

Laserstrahlen können auch eingesetzt werden, um das biologische Objekt in einer Lösung an einer gewünschten Position zu halten. Wenn eine Verformung des biologischen Objekts unter Verwendung eines Laserstrahls hervorgerufen wird, kann durch diesen Laserstrahl auch eine optische Pinzette oder optische Falle für das Objekt erzeugt werden. Dadurch wird es möglich, eine holographische Datenerfassung an einer einzelnen Zelle in Lösung vorzunehmen.

Es wird eine Raman-Spektroskopie vor Setzen eines Reizes und nach oder während dem Setzen des Reizes vorgenommen. Beispielsweise kann die Raman-Spektroskopie sowohl vor dem Setzen des Reizes als auch nach dem Setzen oder während des Setzens des Reizes mit optischen oder weiteren Methoden durchgeführt werden. Auf diese Weise kann beispielsweise eine Veränderung des Zellgeschehens während eines induzierten Stresses im Raman-Spektrum beobachtet werden. Daneben kann durch rechnergestützte Auswertung herkömmlicher 2D-Aufnahmen die Verformung des biologischen Objekts quantitativ bestimmt werden. So können neben der Veränderung des Raman-Spektrums aus der quantitativen Messung der Elastizität auch Informationen zum Vernetzungsgrad von Strukturmolekülen, wie beispielsweise dem Mikrotubuli-Netzwerk oder dem Aktin-Netzwerk, gewonnen werden.

Wenn eine Raman-Spektroskopie durchgeführt wird, kann ein Mess-Laserstrahl, der für die Raman-Spektroskopie auf das biologische Objekt eingestrahlt wird, zwischen verschiedenen Positionen gerastert werden. Es kann eine Rasterung entlang einem Fluidkanal eines Mikrofluidiksystems und/oder zwischen verschiedenen Fluidkanälen des Mikrofluidiksystems erfolgen. Auf diese Weise wird erreicht, dass die durchschnittliche Dauer eines Messprozesses nicht durch die Zeitskala beschränkt ist, die nötig ist, um ein neues biologisches Objekt so zu positionieren, dass daran eine Raman-Spektroskopie durchgeführt werden kann. Ähnlich kann auch ein Strahlengang für die DHMI-Datenerfassung kontrolliert zwischen verschiedenen Lagen verstellt werden. Dies kann insbesondere dann vorteilhaft sein, wenn die verschiedenen biologischen Objekte, an denen parallel Messungen durchgeführt werden sollen, einen Abstand aufweisen, der größer als ein Durchmesser der ObjektWelle für die DHMI-Datenerfassung in einer Objektebene ist. Falls für die entsprechende Messung mehrere Laserstrahlen auf das biologische Objekt eingestrahlt werden, z.B. bei einer Raman-Spektroskopie, bei der das Licht des Anregungslasers für die Raman-Spektroskopie auch das Fallenpotenzial für das biologische Objekt erzeugt, werden die mehreren Laserstrahlen synchron gerastert.

Wenn bei Verfahren und Vorrichtungen nach Ausführungsformen eine Raman-Spektroskopie durchgeführt wird, muss nicht das gesamte Raman-Spektrum erfasst werden. Beispielsweise kann nur ein Teil des Spektrums, z.B. einzelne Spektrallinien, erfasst werden. Dies kann für einen Abgleich mit einer Datenbank, in der charakteristische Spektrallinien des Raman-Spektrums oder deren Verschiebung als Reaktion auf den Reiz abgelegt sind, ausreichend sein. Der Spektralbereich, in dem die Raman-Spektroskopie durchgeführt wird, kann abhängig von den zu charakterisierenden biologischen Objekten gewählt werden.

Der Reiz auf das biologische Objekt muss nicht notwendig unter Verwendung optischer Strahlung ausgeübt werden. Vielmehr können auch Ultraschallwellen oder elektromagnetische Felder, insbesondere Hochfrequenzfelder, eingesetzt werden. Der Reiz kann auch chemisch, beispielsweise durch Gabe von Wirkstoffen oder Medikamenten ausgelöst werden. Der Reiz kann auch mechanisch gesetzt werden, beispielsweise durch Bewegen der Zellen über strukturierte Oberflächen oder durch Absiedlung von Zellen auf Oberflächen.

Bei allen Ausführungsformen kann zusätzlich zu einer Raman-Spektroskopie und vorzugsweise zusätzlich einer DHMI-Datenerfassung auch eine herkömmliche Mikroskopie durchgeführt werden. Beispielsweise kann eine Phasenkontrastmikroskopie, Fluoreszenzmikroskopie oder sonstige Mikroskopie eingesetzt werden, um mikroskopische Beobachtungen des Objekts, das gerade charakterisiert wird, zu ermöglichen.

Bevorzugt wird bei den Ausführungsformen der Erfindung ein Mikrofluidiksystem verwendet. Mit dem Mikrofluidiksystem können biologische Objekte in Lösung gehalten werden. Dies erlaubt es, eine Charakterisierung an lebenden Zellen vorzunehmen. Vorteilhaft wird das zu charakterisierende biologische Objekt, beispielsweise eine Zelle, in dem Mikrofluidiksystem transportiert. Zur Durchführung der Messung an dem biologischen Objekt kann das Objekt dann an eine gewünschte Messposition gebracht werden, beispielsweise unter Verwendung optischer Pinzetten oder Fallen oder eines Ablenkpulses von einem Lasermikrostrahl, mit denen das biologische Objekt aus einem zirkulierenden Fluidstrom entnommen werden kann, oder unter Verwendung mikrofluidischer Ventile oder anderer Stellelemente.

Bei den verschiedenen Ausführungsformen kann vorteilhaft auch die Raman-Spektroskopie und vorzugsweise zusätzlich DHMI-Datenerfassung an dem Objekt in einem Fluidkanal erfolgen. Es kann sowohl die Messung vor Reizsetzung als auch die weitere Messung, die während oder nach Reizsetzung erfolgt, an dem Objekt in dem Fluidkanal erfolgen.

Das Mikrofluidiksystem kann einen geschlossenen Fluidkreislauf aufweisen, in dem biologische Objekte transportiert werden, bis an ihnen die Messung vorgenommen wird. Dadurch kann ein Absinken biologischer Objekte vermindert werden. Die Probe in Lösung kann weitgehend der Charakterisierung zugeführt werden.

Bei den Verfahren und Vorrichtungen nach verschiedenen Ausführungsformen kann nicht nur ein Reiz gesetzt werden, sondern es können mehrere Reize verschiedener Intensität und/oder verschiedener Art gesetzt werden. Es kann die Reaktion des biologischen Objekts auf die verschiedenen Reize unter Verwendung von Raman-Spektroskopie und vorzugsweise zusätzlich von DHMI ermittelt werden, um das biologische Objekt zu charakterisieren. Eine Vorrichtung kann entsprechend mehrere Einrichtungen umfassen, mit denen Reize verschiedener Art gesetzt werden können. Beispielsweise kann eine Einrichtung zum Setzen eines Reizes unter Verwendung optischer Methoden, eine Einrichtung zum Setzen eines Reizes unter Verwendung von Ultraschallwellen und/oder eine Einrichtung zum Setzen eines Reizes unter Verwendung von Hochfrequenzfeldern vorgesehen sein. Auf diese Weise kann das Verhalten des Objekts als Reaktion auf verschiedene Reize ermittelt werden. Es können jeweils extrinsische und/oder intrinsische Änderungen erfasst werden. Bei einer Ausführungsform wird unter Verwendung von sowohl DHMI als auch Raman-Spektroskopie das Verhalten der Zelle nach Setzen von wenigstens zwei verschiedenen Reizen ermittelt, die jeweils gesetzt werden unter Verwendung von einem von Laserstrahlung, Ultraschall oder elektromagnetischen Hochfrequenzfeldern. Eine Deformation einer Zelle kann neben der Änderung der Form und des Volumens zu Proteinumlagerungen führen. Durch Verwendung von Ultraschall kann beispielsweise eine lokale Molekülzusammensetzung verändert und/oder Wärme erzeugt werden. Durch elektromagnetische Hochfrequenzfelder kann eine Oszillation von Molekülen und somit eine intrinsische Veränderung des Objekts erzeugt werden. Durch Datenerfassung mit Raman-Spektroskopie und vorzugsweise zusätzlich DHMI kann ein biologisches Objekt im Hinblick auf seine äußeren Parameter, aber auch im Hinblick auf Vorgänge im Inneren des Objekts charakterisiert werden.

Die Eigenschaften der Raman-Spektroskopie und DHMI erlauben, dass die Charakterisierung von biologischen Objekten weitgehend automatisiert wird. Insbesondere eignen sich die mit der Raman-Spektroskopie gewonnenen Spektren und/oder die Informationen über Form, Volumen und/oder Brechungsindex, die aus DHMI gewonnen werden, für eine weitgehend automatisierte Charakterisierung. Beispielsweise können gewonnene Raman-Spektren und/oder Informationen über Volumen und Form des biologischen Objekts mit einer Datenbank abgeglichen werden, um Zellen automatisch zu sortieren. So können beispielsweise gesunde Zelle von Tumorzellen unterschieden und automatisch zu unterschiedlichen Sammelbehältern gelenkt werden. Bei weiteren Ausführungsformen können bestimmte Zellen, Zellzyklen oder Klone durch Auswertung der Reaktion bei Setzen eines entsprechenden Stimulus erkannt werden.

Die Erfindung wird nachfolgend unter Bezugnahme auf die Zeichnung anhand bevorzugter Ausführungsbeispiele erläutert.

Fig. 1 zeigt eine schematische Darstellung eines Systems zum Charakterisieren und Sortieren biologischer Objekte nach einem Ausführungsbeispiel.

Fig. 2 zeigt eine schematische Darstellung einer Vorrichtung zum Charakterisieren biologischer Objekte nach einem Ausführungsbeispiel.

Fig. 3 zeigt eine schematische Darstellung einer Einrichtung zur Raman-Spektroskopie für eine Vorrichtung nach einem Ausführungsbeispiel.

Fig. 4 zeigt eine schematische Darstellung einer Vorrichtung zum Charakterisieren biologischer Objekte nach einem weiteren Ausführungsbeispiel.

Fig. 5 zeigt eine schematische Darstellung eines Mikrofluidiksystems zur parallelen Durchführung von Messungen bei Vorrichtungen nach einem Ausführungsbeispiel.

Fig. 6 zeigt eine schematische Darstellung einer Vorrichtung zum Charakterisieren biologischer Objekte nach einem weiteren Ausführungsbeispiel.

Fig. 7 zeigt eine schematische Darstellung einer Zelle zur Erläuterung der Wirkungsweise der Vorrichtung von Fig. 6.

Fig. 8 zeigt eine schematische Darstellung einer Vorrichtung zum Charakterisieren biologischer Objekte nach einem weiteren Ausführungsbeispiel.

Fig. 9 ist eine Flussdiagrammdarstellung eines Verfahrens nach einem Ausführungsbeispiel.

Fig. 10 illustriert eine Datenerfassung und Datenverarbeitung bei einem Verfahren nach einem Ausführungsbeispiel.

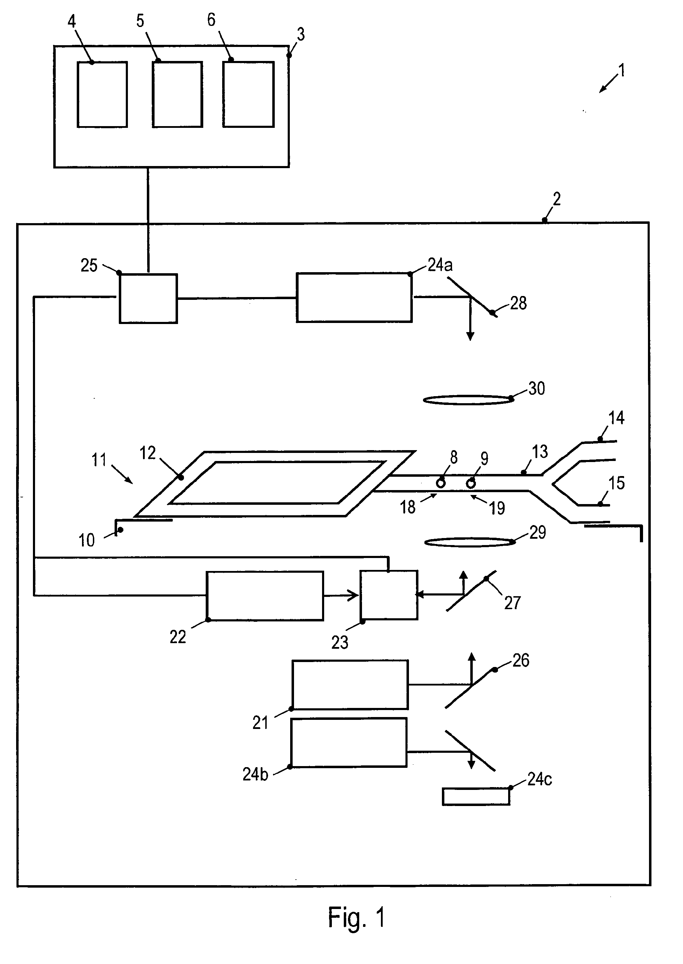

Fig. 1 ist eine schematische Darstellung eines Systems 1 zum Sortieren biologischer Objekte nach einem Ausführungsbeispiel. Das System 1 umfasst eine Vorrichtung 2 zum Charakterisieren biologischer Objekte und eine Recheneinrichtung 3, die mit der Vorrichtung 2 gekoppelt ist. Die Vorrichtung 2 führt eine Raman-Spektroskopie und vorzugsweise zusätzlich eine digitale holographische Mikrointerferometrie (DHMI) an einem biologischen Objekt durch, um dessen Reaktion auf einen Reiz (Stimulus) quantitativ zu ermitteln. Dabei gewonnene Messergebnisse werden über eine Schnittstelle an die Recheneinrichtung 3 ausgegeben werden. Die Recheneinrichtung 3 weist eine Datenbank 4 auf, in der Informationen betreffend das Verhalten verschiedener biologischer Objekte aufweisen. Beispielsweise kann die Datenbank 4 Informationen über ein Raman-Spektrum nach Setzen des Stimulus, Informationen über Verschiebungen von Spektrallinien des Raman-Spektrums als Reaktion auf den Stimulus, Informationen über Form oder Volumen nach Setzen des Stimulus oder Informationen über Änderungen der Form oder Änderungen des Volumens für verschiedene Typen von Zellen (gesunde Zellen oder Tumorzellen), für verschiedene Zellzyklusstadien oder dergleichen umfassen. Die Recheneinrichtung 3 kann abhängig von einem Vergleich der erfassten und verarbeiteten Daten, die beispielsweise eine Änderung eines Raman-Spektrums oder eines Brechungsindex repräsentieren könne, mit einem Schwellenwert oder einem Abgleich der erfassten Daten mit der Datenbank 4 einen Steuerbefehl zum Sortieren des biologischen Objekts an die Vorrichtung 2 ausgeben, Informationen über das Objekt über eine Anzeigeeinrichtung 5 ausgeben oder dem Benutzer verschiedene Optionen für eine weitere Verarbeitung des Objekts anbieten, die der Benutzer über eine Benutzerschnittstelle 6 auswählen kann.

Die Vorrichtung 2 ist eingerichtet, um Zellen berührungslos und schnell zu charakterisieren. Die Vorrichtung 2 weist ein Mikrofluidiksystem 11 auf, das von einem Träger 10 gestützt wird. Das Mikrofluidiksystem 11 umfasst einen geschlossenen Fluidkreislauf 12. Mit geeigneten mikrofluidischen Einrichtungen kann im Betrieb der Vorrichtung 2 ein kontinuierlicher Fluidstrom in dem geschlossenen Fluidkreislauf 12 aufrecht erhalten werden, um das Sedimentieren von biologischen Objekten zu vermeiden, die in einer Lösung in dem Fluidkreislauf 12 transportiert werden. Die Charakterisierung von Zellen erfolgt in dem Mikrofluidiksystem 11. Dies erlaubt es, Messungen an lebenden Zellen oder Zellclustern durchzuführen. Das Mikrofluidiksystem 11 weist einen Fluidkanal 13 auf. Zur Charakterisierung von biologischen Objekten, beispielsweise Zellen, werden die Zellen 8, 9 in dem Fluidkanal 13 positioniert. Auch wenn in Fig. 1 beispielhaft ein separater Fluidkanal 13 dargestellt ist, in den Zellen aus dem geschlossenen Fluidkreislauf 12 beispielsweise mit mikrofluidischen Einrichtungen oder einer optischen Pinzette verschoben werden können, kann der Fluidkanal 13 auch ein Abschnitt des geschlossenen Kreislaufs 12 sein. Auch ein Laserpuls eignet sich, um die Zelle aus einem Laminarstrom in einen Nachbarkanal zu transportieren, wo die Messung durchgeführt wird.

Zur Durchführung einer Charakterisierung wird die Zelle 8 in einem Messbereich 18 und die Zelle 9 in einem Messbereich 19 positioniert. Die Messbereiche 18, 19 sind entlang der Längsachse des Fluidkanals 13 beabstandet. Die Messbereiche 18, 19 können beispielsweise durch den Fokalbereich von Laserstrahlen definiert sein, die eine optische Falle oder optische Pinzette für die Zellen 8 bzw. 9 bilden. Ein Fluidstrom im Fluidkanal 13 kann angehalten werden, wenn eine Messung an den Zellen 8, 9 durchgeführt werden soll. Eine Zentrierung im Kanal kann beispielsweise durch hydrodynamische Fokussierung erfolgen. Eine Positionierung entlang des Kanals erfolgt beispielsweise bei Durchführung einer Raman-Spektroskopie automatisch, wenn die Zellen 8, 9 in den Fokalbereich des zur Durchführung der Raman-Spektroskopie eingestrahlten Laserstrahls gezogen werden.

Während in Fig. 1 eine Konfiguration nach einem Ausführungsbeispiel dargestellt ist, bei der in dem Kanal 12 mehrere Messbereiche 18, 19 in Längsrichtung des Kanals beabstandet sind, können verschiedene weitere Ausgestaltungen bei jeder der hier beschriebenen Ausführungsformen realisiert werden. So kann beispielsweise die Messung jeweils immer nur an einem einzigen Objekt durchgeführt werden, so dass auf die parallele Durchführung von Messungen verzichtet werden kann. Bei einer weiteren Ausführungsform können verschiedene biologische Objekte, an denen eine Messung durchgeführt wird, in Transversalrichtung des Kanals beabstandet sein. Dazu können mehrere biologische Objekte in transversal beabstandeten Teilen eines Laminarstroms in einem Kanal oder in mehreren transversal beabstandeten Laminarströmen positioniert sein. Es können auch mehrere biologische Objekte in transversal beabstandeten Kanälen positioniert sein, wenn die Messung durchgeführt wird.

Die Vorrichtung 2 weist eine Einrichtung 21 zum Erzeugen eines Reizes auf die Zellen 8, 9 auf. Die Einrichtung 21 kann beispielsweise eine Laserquelle, eine Quelle für elektromagnetische Strahlung, insbesondere im Hochfrequenzbereich, oder eine Einrichtung zur Erzeugung von Ultraschallwellen umfassen. Beispielhaft dargestellt ist eine Ausgestaltung, bei der die Einrichtung 21 eine Laserquelle zum Erzeugen eines Mikrostrahls aufweist, der über einen Strahlteiler 26 und eine Linse 29 auf die Zellen 8 bzw. 9 in den Messbereichen 18 bzw. 19 eingestrahlt werden kann. Eine Leistung der Laserquelle 21 kann so gewählt werden, dass durch den Mikrostrahl eine lokale Störung an der Zellmembran oder/und im Zytoplasma der Zellen 8, 9 hervorgerufen wird. Andere Ausgestaltungen der Einrichtung, mit denen unter Verwendung eines Laserstrahls ein Stimulus gesetzt wird, sind gleichfalls möglich. Insbesondere kann die Einrichtung 21 so ausgestaltet sein, dass sie einen Laserstrahl oder mehrere Laserstrahlen mit einer Energiedichte, einem Profil und einer Richtung erzeugen, die eine Verformung der Zelle 8, 9 hervorrufen. Es können auch zwei gegenläufige getaktete Lasermikrostrahlen eingesetzt werden, wobei durch einen Laserpuls oder eine Folge von Laserpulsen eine Deformation der Zelle hervorgerufen wird. Die zwei Strahlen sind dabei so getaktet, dass sie simultan Lichtleistung an das biologische Objekt liefern. Es kann auch ein Anregungsstrahl für die Raman-Spektroskopie gepulst auf das biologische Objekt eingestrahlt werden. Eine Repetitionsrate der Pulse kann variiert werden, um eine Resonanzschwingung des biologischen Objekts anzuregen. Das Auftreten der Resonanz kann durch Beobachtung der 3D-Form, beispielsweise mit DHMI, erkannt und kontrolliert werden. Es kann eine Regelschleife vorgesehen sein, um bei Auftreten der Resonanz die Laserlichtleistung so anzupassen, dass eine Zerstörung des biologischen Objekts vermieden werden kann.

Die Vorrichtung 2 weist eine Einrichtung 22 zum Durchführen einer Raman-Spektroskopie und vorzugsweise zusätzlich eine Einrichtung 24a, 24b, 24c zum Durchführen einer DHMI auf. Mit dieser Einrichtung bzw. diesen Einrichtungen wird das Verhalten der Zelle 8, 9 als Reaktion auf den Reiz ermittelt. Die Vorrichtung 2 des Systems 1 von Fig. 1 umfasst sowohl die Einrichtung 22 zum Durchführen der Raman-Spektroskopie und eine Einrichtung 24a, 24b, 24c zum Durchführen einer DHMI. Je nach Anwendung kann auch nur eine dieser Einrichtungen vorhanden sein. Eine steuerbare Ablenkeinrichtung 23 ist vorgesehen, um einen Anregungsstrahl der Einrichtung 22 zum Durchführen der Raman-Spektroskopie so abzulenken, dass selektiv das Spektrum der Zelle 8 oder der Zelle 9 gemessen wird. Der Anregungsstrahl der Raman-Einrichtung 22 wird über einen Strahlteiler 27 und eine Linse 29, beispielsweise das Objektiv eines Mikroskops, auf die Zelle 8, 9 gelenkt. Eine Steuer- und Auswertelogik 25 ist mit der Raman-Einrichtung 22, der Ablenkeinrichtung 23 und der DHMI-Einrichtung 24a, 24b, 24c gekoppelt, um diese zur Durchführung einer Datenerfassung zu steuern und um die von der Raman-Einrichtung 22 und der DHMI-Einrichtung 24a, 24b, 24c erfassten Daten auszuwerten. Die Raman-Einrichtung 22 und die DHMI-Einrichtung 24a, 24b, 24c führen dabei jeweils eine Datenerfassung vor, bei oder nach Ausüben des Reizes an der entsprechenden Zelle 8, 9 durch, um die Reaktion der Zelle auf den Reiz zu ermitteln. Durch einen Vergleich von vor Setzen des Reizes und nach oder während Setzen des Reizes erfassten DHMI- und Raman-Signalen lassen sich Aussagen zu dem Verhalten der Zellen als Reaktion auf den Reiz gewinnen.

Ähnlich wie Festkörper weisen auch biologische Objekte Schwingungsspektren auf, die beispielsweise abhängig von Zellaktivitäten und Zellzyklusstadien sind. Entsprechend können durch den Reiz verursachte Veränderungen beobachtet werden. Zur Durchführung der Raman-Spektroskopie an lebenden biologischen Objekten kann die Raman-Einrichtung 22 beispielsweise einen Diodenlaser mit einer Wellenlänge von 785 nm umfassen. Der Laserstrahl kann auf die Zellen 8, 9 fokussiert werden. Bei dieser Wellenlänge werden die Zellen und Bakterien nicht geschädigt. Die gestreuten Ramansignale werden mittels eines Spektrometers gemessen. Das Spektrometer kann beispielsweise mit einem für das nahe Infrarot optimierten CCD-Sensor ausgestattet sein.

Eine Verstellung des für die Ramanspektroskopie auf die Zellen eingestrahlten Laserstrahls kann mit der Ablenkeinrichtung 23 auch so erfolgen, dass das biologische Objekt, beispielsweise die Zelle, an mehreren Punkten abgetastet wird. Dadurch kann für jeden der verschiedenen Messpunkte ein Ramanspektrum aufgenommen werden. Aus den unterschiedlichen Spektren lassen sich Rückschlüsse auf die chemische oder biochemische Zusammensetzung des biologischen Objekts nach Setzen des Reizes ziehen. Durch Rasterung über das biologische Objekt kann diese Information auch ortsaufgelöst ermittelt werden.

Die Raman-Einrichtung 22 kann so ausgestaltet sein, dass eine Position eines Fokus des Anregungsstrahls für die Durchführung der Raman-Spektroskopie in allen drei Raumrichtungen eingestellt werden kann. Wenn die Vorrichtung 2 ein optisches Mikroskop beinhaltet, ist die Raman-Einrichtung 22 vorteilhaft so ausgestaltet, dass die Position des Fokus des Anregungsstrahls für die Durchführung der Raman-Spektroskopie unabhängig von einem Fokus des Mikroskopstrahlengangs kontrolliert werden kann.

Die Erfassung von Raman-Spektren kann derart erfolgen, dass vor oder nach Erfassen der Raman-Spektren an einem biologischen Objekt weiterhin automatisch ein Raman-Spektrum eines Substrats erfasst wird, an dem das biologische Objekt angeordnet ist. Dieses Hintergrund-Spektrum kann verwendet werden, um rechnerisch Signalanteile, die durch das Substrat hervorgerufen werden, von den bei der Raman-Spektroskopie an den biologischen Objekten gewonnenen Daten abzuziehen. Die Lage und Höhe von Peaks im Hintergrund-Spektrum kann mit der Lage und Höhe von Peaks in den am biologischen Objekt erfassten Raman-Spektren verglichen werden, um einen multiplikativen Faktor zu ermitteln, der das Gewicht des Hintergrundsignals in den am biologischen Objekt erfassten Raman-Spektren angibt und der bei der rechnerischen Subtraktion des Hintergrund-Spektrums berücksichtigt wird. Beispielsweise kann das Hintergrund-Spektrum mit dem multiplikativen Faktor, der das Gewicht angibt, multipliziert werden, bevor es von den an dem biologischen Objekt erfassten Raman-Spektren subtrahiert wird. Die Subtraktion des Hintergrundspektrums kann insbesondere vorteilhaft eingesetzt werden, wenn das biologische Objekt unmittelbar auf einem festen Substrat angeordnet ist.

Die DHMI-Einrichtung ist so eingerichtet, dass ein Laserstrahl in zwei Teilstrahlen geteilt wird, die eine Objektwelle und eine dazu phasenkohärente Referenzwelle bilden. Die Objektwelle und Referenzwelle können über geeignete optische Komponenten so gelenkt werden, dass die Objektwelle auf das Objekt gelenkt wird, wobei an dem Objekt gestreute oder reflektierte Signale der Objektwelle auf einen flächigen Bildsensor, beispielsweise einen CCD-Sensor, treffen. Die Referenzwelle trifft auf den flächigen Bildsensor, ohne an dem Objekt reflektiert oder gestreut zu werden. In Fig. 1 weist die DHMI-Einrichtung eine schematisch dargestellte Komponente 24a auf, die die Objektwelle ausgibt. Die Objektwelle wird über einen Strahlteiler 28 und die Kondensor-Linse 30 auf eine der Zellen 8, 9 oder gleichzeitig auf beide Zellen 8, 9 gelenkt. Von einer Komponente 24b wird die Referenzwelle über einen Strahlteiler auf einen flächigen Bildsensor 24c, beispielsweise einen CCD-Sensor gelenkt. Das durch die Phasendifferenzen zwischen Objektwelle und Referenzwelle an dem Bildsensor 24c resultierende Interferenzmuster erlaubt eine Rekonstruktion der Form des biologischen Objekts.

Die DHMI-Einrichtung mit den Komponenten 24a, 24b, 24c bietet eine berührungslose, markerfreie und quantitative Phasenkontrast-Bildgebung. Aus einem einzigen mit der DHMI-Einrichtung 24a, 24b, 24c erfassten Hologramm können verschiedene Objektebenen rekonstruiert werden. Neben einer markerfreien Topographie- bzw. Morphologieanalyse ist auch eine minimal-invasive dynamische Detektion von Verformungen und Bewegungen lebender Zellen in drei Dimensionen möglich. Durch rechnerische Auswertung des von dem Bildsensor 24c erfassten Signals kann eine digitale Nachfokussierung vorgenommen werden. So wird Stabilität auch dann erreicht, wenn sich die Zelle in Tiefenrichtung bewegt. Verformungen des biologischen Objekts können so in drei Dimensionen zur Zellcharakterisierung erfasst werden.

Mit der Vorrichtung 2 kann somit die Reaktion eines biologischen Objekts im Hinblick auf Veränderungen der Topographie oder Morphologie sowie im Hinblick auf Veränderungen des Ramanspektrums untersucht werden. Die Messung der Reaktion auf einen bekannten Reiz erlaubt es, das Verhalten des biologischen Objekts sowohl im Hinblick auf Änderungen der Morphologie bzw. Topographie als auch im Hinblick auf Änderungen des Raman-Spektrums zu erfassen. Durch einen Abgleich mit einer entsprechenden Datenbank kann beispielsweise ermittelt werden, um welchen Zelltyp es sich handelt, in welchem Zellzyklenstadium sich dieser befindet etc. Sowohl die Raman-Spektroskopie als auch die DHMI sind schnelle Messmethoden, da sie in einer einzigen Aufnahme die wesentlichen Eigenschaften, d.h. das Spektrum bzw. Hologramm, erfassen können. Bei Ausführungsformen wird nur ein Teil des Raman-Spektrums erfasst, um einen Abgleich mit einer Datenbank vornehmen zu können. Beispielsweise können einzelne Linien eines Raman-Spektrums erfasst werden, anhand derer ein Abgleich mit bekannten Datensätzen erfolgen kann. Auf diese Weise kann eine weitere Erhöhung des Durchsatzes erreicht werden. Die relevanten Linien können einmal vor und ein weiteres Mal während oder nach Reizsetzung gemessen werden. Es können Differenzen der spektralen Gewichte, d.h. der Peaks im erfassten Spektrum, für die relevanten Linien des Spektrums ermittelt werden, um anhand der Differenzen das biologische Objekt zu charakterisieren.

Da die Charakterisierung bei der in Fig. 1 dargestellten Vorrichtung 2 markerfrei und ohne Zerstörung des biologischen Objekts abläuft, können die biologischen Objekte weiter verarbeitet werden. Dazu kann beispielsweise von der Steuer- und Auswertelogik 25 oder von der Recheneinrichtung 3 ein Signal bereitgestellt werden, nach dem die Zelle 8, 9 selektiv in einen von mehreren Ausgangskanälen 14, 15 des Mikrofluidiksystems 11 sortiert wird.

Die Blockdiagrammdarstellung der verschiedenen Komponenten der Vorrichtung 2 ist als schematische Darstellung zu betrachten. Beispielsweise können Komponenten der Raman-Einrichtung 22, 23 auch so vorgesehen sein, dass der Anregungsstrahl über die Linse 30 eingekoppelt wird. Alternative Ausgestaltungen der Einrichtung 21, mit der der Stimulus gesetzt wird, sind ebenfalls möglich.

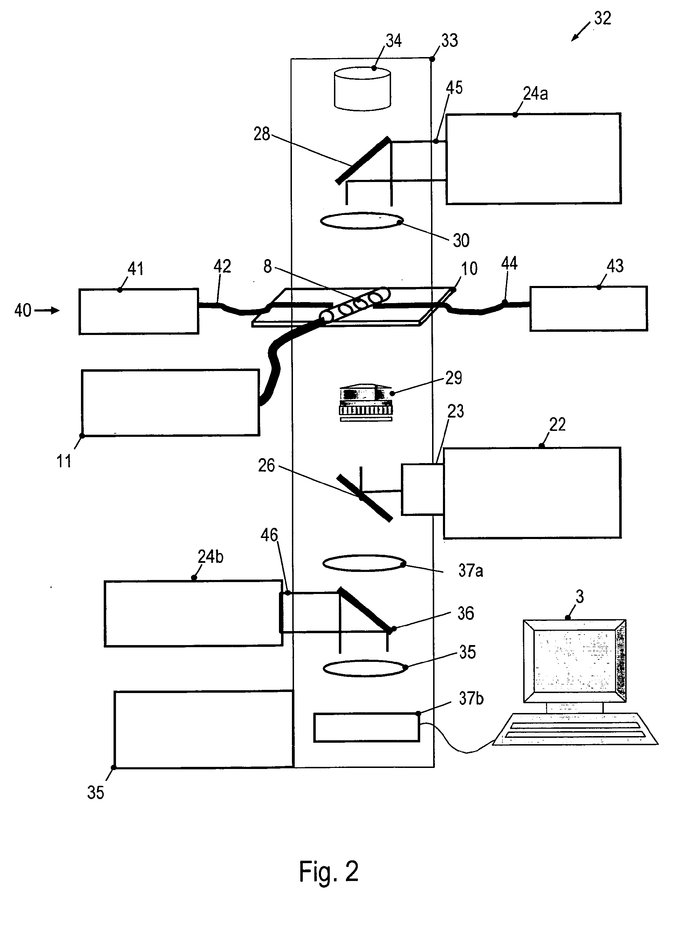

Fig. 2 ist eine schematische Darstellung einer Vorrichtung 32 zum Charakterisieren einer Zelle nach einem weiteren Ausführungsbeispiel. Komponenten der Vorrichtung 32, die in ihrer Funktion und/oder Ausgestaltung Komponenten der Vorrichtung 2 entsprechen, sind mit denselben Bezugszeichen wie in Fig. 1 bezeichnet.

Die Vorrichtung weist neben den Modulen 24a und 24b der DHMI-Einrichtung, die die Objektwelle und Referenzwelle für die DHMI bereitstellen, und der Raman-Einrichtung 22 Komponenten eines herkömmlichen Mikroskopsystems auf. Die Objekt- und Referenzwelle für die DHMI-Datenerfassung und der Anregungsstrahl für die Raman-Spektroskopie können in den Strahlengang des Mikroskops eingekoppelt werden. Das Mikroskop weist eine Beleuchtungseinrichtung 34 auf. Weitere aus der Mikroskopie bekannte Komponenten, die schematisch bei 35 dargestellt sind, können vorgesehen sein. Eine flächiger Bildsensor 36, beispielsweise einen CCD-Sensor, kann sowohl zur Bilderfassung für die herkömmliche Mikroskopie an den biologischen Objekten als auch zur Datenerfassung bei der DHMI dienen. Tubuslinsen 37a, 37b können in einem Mikroskopkorpus 33 vorgesehen sein.

Die Vorrichtung 32 weist sowohl eine Einrichtung 24a, 24b, 38 zur DHMI-Datenerfassung als auch eine Einrichtung 22 zur Erfassung eines Raman-Spektrums auf. Ein Objektstrahl 45 für die DHMI wird über den Strahlteiler in den Strahlengang des Mikroskops eingekoppelt und über die Kondenser-Linse 30 auf einen Fluidkanal eines nur schematisch dargestellten Mikrofluidiksystems 11 gelenkt. In dem Fluidkanal ist ein biologisches Objekt 8, beispielsweise eine Zelle, zur Charakterisierung platziert. Ein Referenzstrahl 46 für die DHMI wird über einen Strahlteiler 36 und eine Tubuslinse 37b auf den Bildsensor 38 gelenkt. Ein Anregungsstrahl für die Raman-Spektroskopie wird über eine steuerbare Ablenkeinrichtung 23 und einen Strahlteiler 26 in den Strahlengang des Mikroskops eingekoppelt. Über das Objektiv 29 wird der Anregungsstrahl auf das biologische Objekt 8 fokussiert. Die steuerbare Ablenkeinrichtung 23 erlaubt die örtlich aufgelöste Erfassung von Raman-Spektren an einem biologischen Objekt, beispielsweise an einer Zelle. Optional kann eine Rasterung über verschieden platzierten Objekten vorgesehen sein, um zeitsequentiell Raman-Spektrallinien von Objekten zu erfassen. An dem Objekt gestreutes Licht wird zu einem Spektrometer geführt. Beispielsweise kann das Streulicht über den Strahlteiler 26 zu einem Raman-Spektrometer geführt werden, das in der Raman-Einrichtung 22 vorgesehen ist.

Die Vorrichtung 32 weist eine Einrichtung 40 zum Ausüben eines Reizes auf das biologische Objekt 8 auf. Die Einrichtung umfasst zwei Fasern 42, 44, mit denen ein optisches Signal hoher Intensität zu einem Messbereich des Fluidkanals geführt werden kann, in dem das biologische Objekt zur Durchführung der Messung positioniert ist. Die Fasern 42, 44 sind mit einer oder mehreren Quellen 41, 43 für das optische Signal gekoppelt. Es kann ein Laser 41 vorgesehen sein, wobei der Laserstrahl über geeignete optische Komponenten in die erste Faser 42 und über optische Komponenten 43 in die zweite Faser 44 eingekoppelt werden kann. Die optischen Fasern 42, 44 können so angeordnet sein, dass die aus ihren Enden austretenden Strahlen im Wesentlichen gegenläufig sind. Auf diese Weise kann optisch ein Reiz auf das biologische Objekt 8 ausgeübt werden. Der Reiz kann durch einen kurzen Laserpuls ausgeübt werden, wobei vor dem Setzen des Laserpulses und/oder während dem Laserpuls und/oder nach Ende des Laserpulses ein Raman-Spektrum und ein DHMI-Bild des Objekts 8 erfasst wird. Der Reiz kann auch über einen längeren Zeitraum ausgeübt werden, um beispielsweise eine länger andauernde Verformung des Objekts 8 hervorzurufen. Ein Raman-Spektrum und ein DHMI-Bild des Objekts 8 können erfasst werden, während Licht von den Enden der Fasern 42, 44 auf das Objekt eingestrahlt wird. Der optische Reiz kann auch gesetzt werden, indem eine pulsierende Kraft auf das biologische Objekt ausgeübt wird, die das biologische Objekt in Resonanzschwingungen versetzt. Entsprechende Datenerfassungen können auch vorgenommen werden, bevor der Reiz gesetzt wird oder wenn die Einstrahlung von Licht von den Enden der Fasern 42, 44 beendet wurde.

Anhand des Raman-Spektrums und der DHMI-Daten, die vor Reizsetzung und während oder nach Reizsetzung erfasst werden, kann das Objekt 8 charakterisiert werden. Dazu kann beispielsweise ein Abgleich mit einer Datenbank erfolgen, um einen Zelltyp oder ein Zellzyklenstadium zu ermitteln. Falls kein passender Eintrag für die erfassten Daten ermittelt werden kann, kann die Datenbank um einen Eintrag erweitert werden, in dem charakteristische Eigenschaften des gemessenen Raman-Spektrums und der aus den DHMI-Daten rekonstruierten Information über Volumen und Form des Objekts 8 einem Objekttyp zugeordnet werden. So wird die Datenbasis für die automatische Charakterisierung erstellt bzw. erweitert.

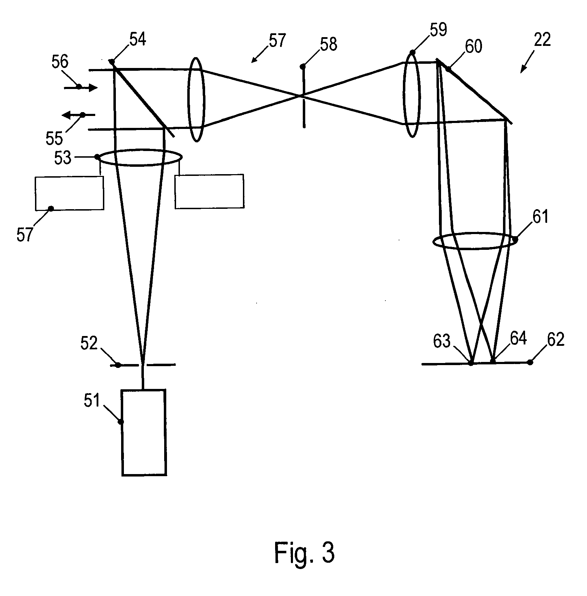

Fig. 3 ist eine schematische Darstellung einer Raman-Einrichtung, die als Einrichtung zum Erfassen eines Raman-Spektrums bei den Vorrichtungen nach den verschiedenen Ausführungsbeispielen verwendet werden kann.

Die Raman-Einrichtung 22 umfasst eine Laserquelle 51. Über eine Lochblende 52, eine Linse 53 und einen Strahlteiler 54 wird ein Ausgangsstrahl der Laserquelle 51 als Anregungsstrahl 55 für die Raman-Streuung zu dem biologischen Objekt gelenkt. Streulicht 56 wird über den Strahlteiler 54, ein Raman-Kantenfilter 57 und eine Lochblende 58 zu einem Raman-Spektrometer gelenkt. Das Raman-Spektrometer umfasst ein Gitter 60 und einen eindimensionalen oder flächigen Bildsensor 62, beispielsweise einen CCD-Sensor. Zwischen der Lochblende 38 und dem Gitter 60 sowie zwischen dem Gitter 60 und dem Bildsensor 62 sind Linsen 59 bzw. 61 vorgesehen. Das von dem Gitter 60 in seine Spektralkomponenten aufgespaltete Streulicht wird so auf verschiedene Positionen 63, 64 an dem Bildsensor 62 gelenkt und vorteilhaft dort fokussiert. An dem Bildsensor 62 werden somit die unterschiedlichen Spektrallinien des Raman-Spektrums erfasst. Bei einer Rasterung des Anregungsstrahls 55 über verschiedene Punkte der Objektoberfläche können zeitsequentiell mehrere Raman-Spektren erfasst werden, die unterschiedlichen Punkten oder Bereichen an der Objektoberfläche zugeordnet sind.

Die Raman-Einrichtung 22 kann so ausgestaltet sein, dass eine Position eines Fokus des Anregungsstrahls 55 in allen drei Raumrichtungen kontrolliert bewegt werden kann. Die Raman-Einrichtung 22 kann so ausgestaltet sein, dass die Position des Fokus des Anregungsstrahls 55 für die Raman-Spektroskopie unabhängig von einem Fokus des Strahlengangs eines optischen Mikroskops der Vorrichtung kontrolliert werden kann, in der die Raman-Einrichtung 22 verwendet wird. Dazu kann beispielsweise eine Positioniereinrichtung 57 vorgesehen sein, die mit der Linse 53 gekoppelt ist, um diese in drei orthogonalen Richtungen (x, y, z) zu verschieben. Auf diese Weise kann die Position des Fokus des Anregungsstrahls 55 kontrolliert werden.

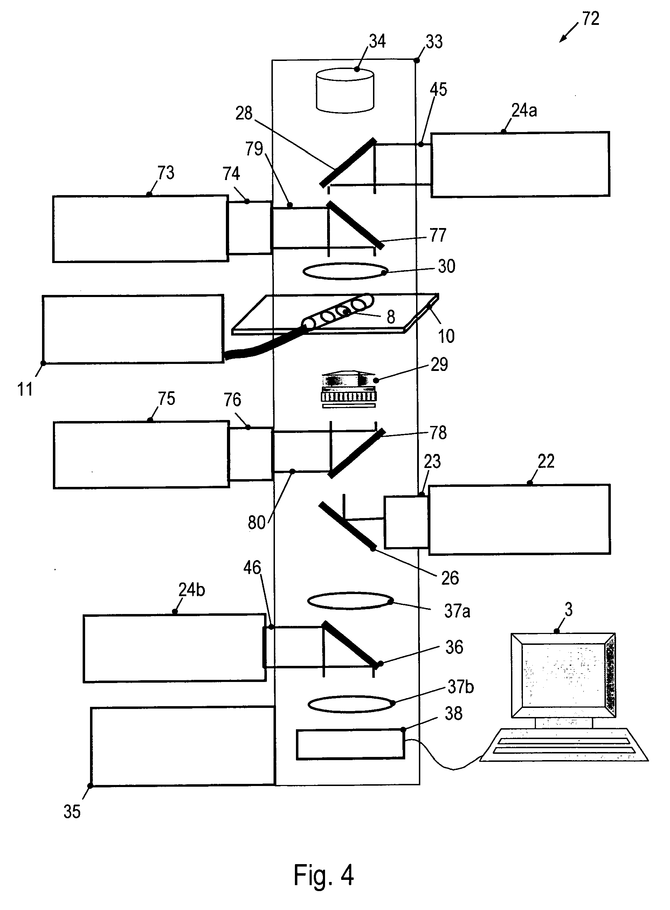

Fig. 4 ist eine schematische Darstellung einer Vorrichtung 72 zum Charakterisieren einer Zelle nach einem weiteren Ausführungsbeispiel. Komponenten der Vorrichtung 72, die in ihrer Funktion und/oder Ausgestaltung Komponenten der Vorrichtung 2 von Fig. 1 oder Komponenten der Vorrichtung 32 von Fig. 2 entsprechen, sind mit denselben Bezugszeichen wie in Fig. 1 bzw. Fig. 2 bezeichnet.

Bei der Vorrichtung 72 wird ein Reiz auf ein biologisches Objekt unter Verwendung optischer Strahlung erzeugt. Dabei wird darauf verzichtet, das Laserlicht unter Verwendung von optischen Fasern bis zu einem Messbereich zu führen. Die Einrichtung zum Erzeugen eines Reizes weist eine Komponente 73 auf, die einen Laserstrahl 79 an eine steuerbare Ablenkeinrichtung 74 ausgibt. Über die Ablenkeinrichtung 74 und einen Strahlteiler 77 wird der Laserstrahl zu der Kondenser-Linse 30 gelenkt. Die Einrichtung zum Erzeugen eines Reizes weist eine Komponente 75 auf, die einen weiteren Laserstrahl 80 an eine steuerbare Ablenkeinrichtung 76 ausgibt. Über die Ablenkeinrichtung 76 und einen Strahlteiler 78 wird der weitere Laserstrahl 80 zu dem Objektiv 29 gelenkt. Die Komponenten 73, 75 können beispielsweise Enden von optischen Fasern sein, mit denen Laserlicht von einem Laser zu den Ablenkeinrichtungen 74, 76 geführt wird. Alternativ kann der Laserstrahl über geeignete Umlenkspiegel 73, 75 zu den Ablenkeinrichtungen 74, 76 geführt werden. Die steuerbaren Ablenkeinrichtungen 74, 76 können eine verstellbares Element, beispielsweise einen mit einem Motor verstellbaren Umlenkspiegel, oder ein elektrooptisches Bauteil, beispielsweise einen räumlichen Lichtmodulator (SLM) umfassen. Die Ablenkeinrichtungen 74, 76 erlauben es, die Laserstrahlen 79, 80 über verschiedene Positionen zu rastern. Dadurch können beispielsweise Reize auf mehrere biologische Objekte, beispielsweise mehrere Zellen, ausgeübt werden, die entlang der Längsachse eines Fluidkanals oder in unterschiedlichen Fluidkanälen des Mikrofluidiksystems positioniert sind. Dadurch wird erreicht, dass mehrere biologische Objekte parallel charakterisiert werden können.

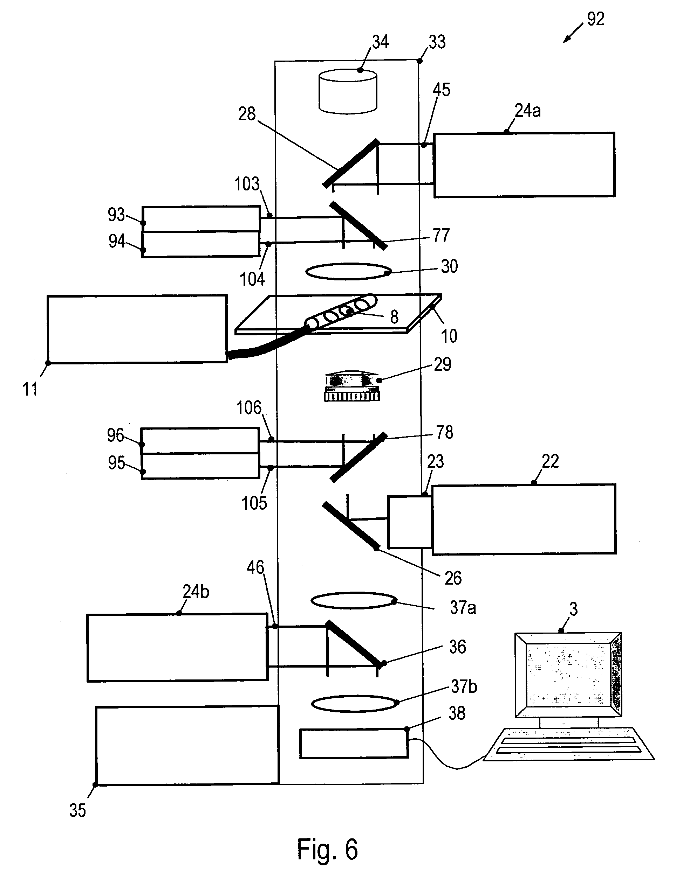

Die Leistung und das Profil der Laserstrahlen 79 und 80 können so gewählt werden, dass eine Verformung des biologischen Objekts 8 hervorgerufen wird. Bei einer Ausgestaltung kann die Leistung und das Profil der Laserstrahlen 79 und 80 so gewählt werden, dass sie auf das biologische Objekt Kräfte derart ausüben, dass das Objekt in der Propagationsrichtung der Strahlen 79 und 80, d.h. in Fig. 6 in vertikaler Richtung, zusammengedrückt wird. Bei einer weiteren Ausgestaltung kann die Leistung und das Profil der Laserstrahlen 79 und 80 so gewählt werden, dass sie auf das biologische Objekt Kräfte derart ausüben, dass das Objekt in der Propagationsrichtung der Strahlen 79 und 80, d.h. in Fig. 6 in vertikaler Richtung, gedehnt wird.

Die Ablenkeinrichtungen 74, 76 werden so gesteuert, dass die Laserstrahlen 79 und 80 synchron über die verschiedenen Positionen gerastert werden. Auf diese Weise können die aus der Technik bekannten Reize, beispielsweise Verformung durch Druck oder Dehnung entlang der Propagationsrichtung der gegenläufigen Strahlen 79 und 80, auch bei Rasterung über mehrere Positionen realisiert werden. Die Rasterung des Anregungsstrahls der Raman-Einrichtung 22 kann vorteilhaft ebenfalls mit der gleichen Rate zwischen den verschiedenen Messbereichen wechseln wie die Strahlen 79, 80 zum Ausüben des Reizes. Jedoch ist es nicht erforderlich, dass der Anregungsstrahl der Raman-Einrichtung 22 zeitgleich mit den Strahlen 79, 80 auf ein zu charakterisierendes biologisches Objekt auftrifft.

Eine Ausgestaltung der Vorrichtung zum Charakterisieren von biologischen Objekten, bei der Laserstrahlen zum Erzeugen des Reizes nicht unter Verwendung von optischen Fasern bis an einen Fluidkanal herangeführt werden, erlaubt eine einfache und schnelle Rasterung der Laserstrahlen. Der Aufbau der Vorrichtung kann vereinfacht werden.

Während in Fig. 4 Ablenkeinrichtungen 74, 76 dargestellt sind, können bei weiteren Ausführungsformen die Ablenkeinrichtungen 74, 76 auch weggelassen werden.

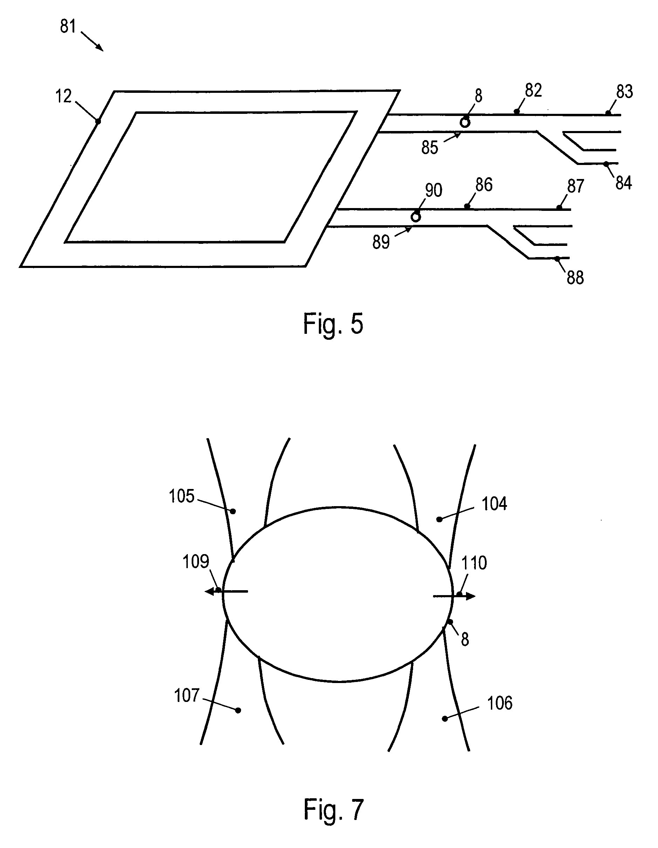

Fig. 5 ist eine schematische Darstellung eines Mikrofluidiksystems 81, bei dem Messungen parallel in mehreren Fluidkanälen erfolgen können. Ein Mikrofluidiksystem mit der in Fig. 5 dargestellten Ausgestaltung kann beispielsweise dann eingesetzt werden, wenn die Strahlen zum Ausüben des Reizes oder der Anregungsstrahl für die Raman-Spektroskopie gerastert werden können.

Das Mikrofluidiksystem 81 weist einen geschlossenen Kreislauf 12 und mehrere Fluidkanäle 82, 86 auf, in denen Messungen an biologischen Objekten durchgeführt werden können. Abhängig von einem Resultat der Charakterisierung kann ein biologisches Objekt 8, das während der Datenerfassung zur Charakterisierung in einem Messbereich 85 in dem Fluidkanal 82 angeordnet ist, selektiv in einen von mehreren Ausgangskanälen 83, 84 sortiert werden. Ähnlich kann abhängig von einem Resultat der Charakterisierung ein biologisches Objekt 90, das während der Datenerfassung zur Charakterisierung in einem Messbereich 89 in dem Fluidkanal 86 angeordnet ist, selektiv in einen von mehreren Ausgangskanälen 87, 88 sortiert werden.

Wenn optische Strahlung, die zum Ausüben des Reizes und/oder zum Messen einer Reaktion auf den Reiz eingesetzt wird, zwischen den Messbereichen 85 und 89 in den verschiedenen Fluidkanälen gerastert werden kann, kann eine Charakterisierung biologischer Objekte parallel in den zwei Fluidkanälen 82 und 86 erfolgen.

Fig. 6 ist eine schematische Darstellung einer Vorrichtung 92 zum Charakterisieren einer Zelle nach einem weiteren Ausführungsbeispiel. Komponenten der Vorrichtung 92, die in ihrer Funktion und/oder Ausgestaltung Komponenten der Vorrichtung 2 von Fig. 1, Komponenten der Vorrichtung 32 von Fig. 2 oder Komponenten der Vorrichtung 72 von Fig. 4 entsprechen, sind mit denselben Bezugszeichen wie in Fig. 1, Fig. 2 bzw. Fig. 4 bezeichnet.

Bei der Vorrichtung 92 wird ein Reiz auf ein biologisches Objekt unter Verwendung optischer Strahlung erzeugt. Dabei werden ähnlich wie bei der Vorrichtung 72 von Fig. 4 Laserstrahlen zum Ausüben des Reizes über die Linse 30 bzw. das Objektiv 29 auf das biologische Objekt 8 eingestrahlt. Die Einrichtung zum Erzeugen eines Reizes weist eine Komponente 93 auf, die einen Laserstrahl 103 ausgibt, der über einen Strahlteiler 77 zu der Linse 30 gelenkt wird. Eine weitere Komponente 95 ist vorgesehen, die einen Laserstrahl 105 ausgibt, der über einen Strahlteiler 78 zu dem Objektiv 80 gelenkt wird. Dabei sind die Komponenten 93, 95 so eingerichtet, dass die Laserstrahlen 103 und 106 als erste optische Pinzette für ein biologisches Objekt 8 wirken. Die Einrichtung zum Erzeugen des Reizes weist auch Komponenten 94, 96 auf, mit denen eine zweite optische Pinzette für dasselbe biologische Objekt 8 erzeugt wird. Die Komponente 94 gibt einen Laserstrahl 104 aus, der über den Strahlteiler 77 zu der Linse 30 gelenkt wird. Die Komponente 96 gibt einen Laserstrahl 106 aus, der über den Strahlteiler 78 zu dem Objektiv 29 gelenkt wird. Die verschiedenen Komponenten 93-96 können von derselben Laserlichtquelle gespeist werden.

Die Einrichtung zum Erzeugen des Reizes kann so eingerichtet werden, dass die von den Laserstrahlen 103 und 105 erzeugte optische Pinzette und die von den Laserstrahlen 104 und 106 erzeugte weitere optische Pinzette einen Abstand aufweisen, der näherungsweise gleich einem Durchmesser des biologischen Objekts ist, oder auf einen solchen Abstand einstellbar sind. Auf diese Weise können auf das biologische Objekt Kräfte ausgeübt werden, die zu einer Dehnung des biologischen Objekts führen.

Fig. 7 zeigt beispielhaft diese Wirkung eines Paars optischer Pinzetten. Die Fokalbereiche der Paars von Laserstrahlen 105, 107 und des Paars von Laserstrahlen 104, 106 sind voneinander beabstandet. Aufgrund der Dipol-Fallenwirkung werden Zellenbereiche in die Volumenbereiche mit maximaler Lichtenergie gezogen. Werden die beiden optischen Pinzetten auf einen geeigneten Abstand eingestellt, beispielsweise durch eine laterale Bewegung der Pinzetten voneinander weg, kann eine Dehnung des biologischen Objekts hervorgerufen werden, wie mit den Pfeilen 109 und 110 dargestellt ist.

Auch bei der Vorrichtung 92 können (in Fig. 6 nicht dargestellte) Ablenkeinrichtungen vorgesehen sein, um eine Verstellung eines der Paare 103, 105 oder 104, 106 von Laserstrahlen oder beider Paare von Laserstrahlen zu erlauben. Durch Verstellen wenigstens eines der Paare von Laserstrahlen, die eine optische Pinzette bilden, ist die Vorrichtung 92 so konfigurierbar, dass sie eine Formänderung auch an biologischen Objekten mit deutlich verschiedenen Abmessungen hervorrufen kann. Durch eine gemeinsame Rasterung beider optischer Pinzetten kann die Rate, mit der biologische Objekte charakterisiert werden können, erhöht werden.

Während Ausführungsbeispiele detailliert beschrieben wurden, bei denen eine Durchlichtkonfiguration für die DHMI-Datenerfassung eingesetzt wird, kann bei jeder der Ausführungsformen auch eine Auflichtkonfiguration verwendet werden.

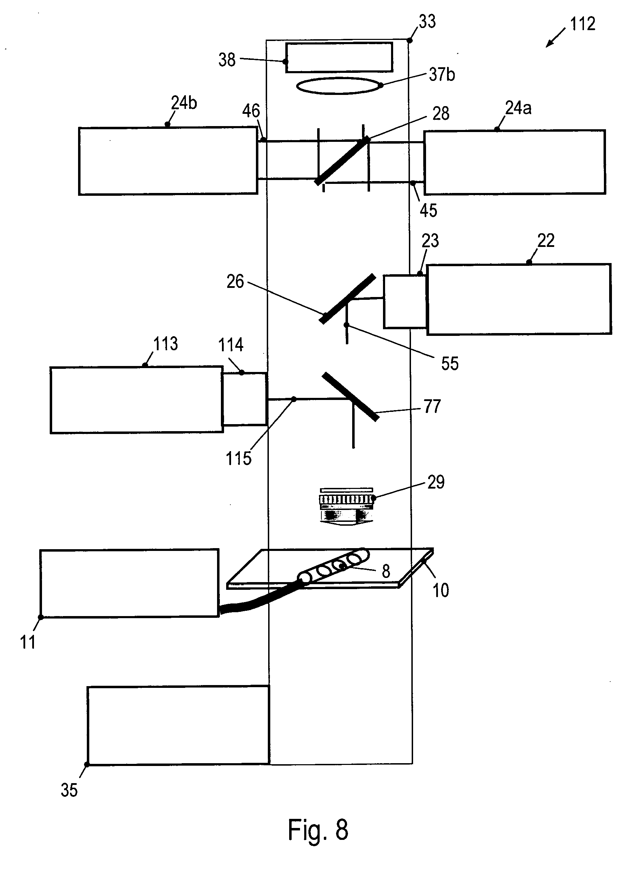

Fig. 8 ist eine schematische Darstellung einer Vorrichtung 112 zum Charakterisieren einer Zelle nach einem weiteren Ausführungsbeispiel. Komponenten der Vorrichtung 112, die in ihrer Funktion und/oder Ausgestaltung Komponenten der Vorrichtung 2 von Fig. 1, Komponenten der Vorrichtung 32 von Fig. 2, Komponenten der Vorrichtung 72 von Fig. 4 oder Komponenten der Vorrichtung 92 von Fig. 6 entsprechen, sind mit denselben Bezugszeichen wie in Fig. 1, Fig. 2, Fig. 4 bzw. Fig. 6 bezeichnet.

Die Vorrichtung 112 weist eine Einrichtung zur DHMI-Datenerfassung mit einer Quelle 24a für die Objektwelle 45 und einer Quelle 24b für die Referenzwelle 46 auf. Das aus Überlagerung resultierende Interferenzmuster wird an dem flächigen Bildsensor 38 erfasst. Die Vorrichtung 112 weist weiterhin eine Einrichtung 22 zum Durchführen der Raman-Spektroskopie auf. Die Vorrichtung 112 weist eine Einrichtung 113 zum Erzeugen eines Laserpulses oder mehrerer Laserpulse auf. Eine steuerbare Ablenkeinrichtung 114 kann optional vorgesehen sein, damit der Laserpuls bzw. die Laserpulse selektiv auf eines von mehreren verschiedenen biologischen Objekten oder auf verschiedene Abschnitte eines biologischen Objekts gerichtet werden kann bzw. können. Mit dem von der Einrichtung 113 erzeugten Mikrostrahl kann beispielsweise eine lokale Störung an der Zellmembran und/oder im Zytoplasma einer Zelle erzeugt werden. Die Reaktion der Zelle auf die Störung kann zum Charakterisieren der Zelle erfasst werden. Dazu kann, wie unter Bezugnahme auf Fig. 1-7 beschrieben wurde, eine DHMI-Datenerfassung und eine Raman-Spektroskopie durchgeführt werden, bevor die Störung erzeugt wurde. Eine weitere DHMI-Datenerfassung und eine weitere Raman-Spektroskopie werden durchgeführt, während die Störung erzeugt wird oder nachdem die Störung erzeugt wurde.

Unter Verwendung der Vorrichtungen können Veränderungen intrinsischer Eigenschaften von biologischen Objekten als Reaktion auf einen Stimulus beobachtet werden. Abhängig von der Veränderung der intrinsischen Eigenschaften kann das biologische Objekt charakterisiert werden. Da Reizsetzung und Beobachtung zerstörungsfrei vorgenommen werden können, kann das biologische Objekt zerstörungsfrei charakterisiert und anschließend einer weiteren Manipulation oder Verwendung unterzogen werden.

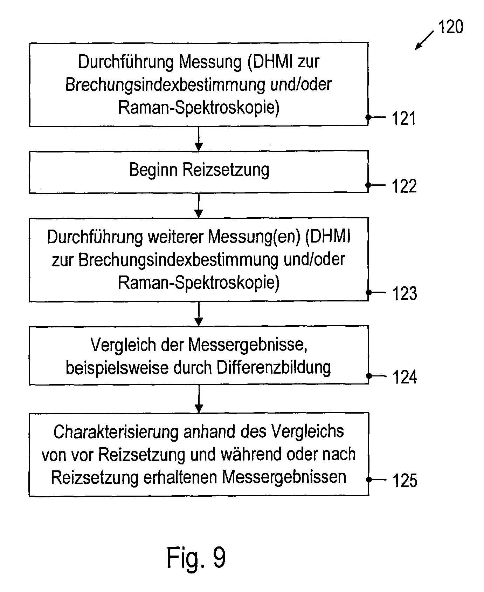

Fig. 9 ist eine Flussdiagrammdarstellung eines Verfahrens 120 nach einem Ausführungsbeispiel. Das Verfahren kann mit der Vorrichtung oder dem System nach einem der unter Bezugnahme auf Fig. 1-8 beschriebenen Ausführungsbeispiele durchgeführt werden.

Bei Schritt 121 wird eine Messung an einem biologischen Objekt durchgeführt. Dabei wird ein Raman-Spektrum erfasst und vorzugsweise zusätzlich eine DHMI-Messung zur Bestimmung eines Brechungsindex durchgeführt. Die Messung bei 121 erfolgt vor Reizsetzung.

Bei Schritt 122 wird ein Reiz gesetzt. Zur Reizsetzung können unterschiedliche bereits beschriebene Methoden eingesetzt werden, die beispielsweise die Verwendung von Laserlicht, elektromagnetischen Wechselfeldern, elektromagnetischer Strahlung, Ultraschall, oder dergleichen beinhalten können.

Bei Schritt 123 kann während oder nach der Reizsetzung wenigstens eine weitere Messung an demselben biologischen Objekt durchgeführt werden, an dem die Messung bei Schritt 121 vorgenommen wurde. Dabei wird eine weitere DHMI-Messung zur Bestimmung des Brechungsindex durchgeführt und/oder ein weiteres Raman-Spektrum erfasst.

Bei Schritt 124 werden die bei 121 und 123 erhaltenen Messergebnisse verglichen. Dazu kann beispielsweise eine Differenz zwischen dem bei 121 erhaltenen Messergebnis und dem bei 123 erhaltenen Messergebnis gebildet werden. Falls bei 121 und 123 jeweils ein Raman-Spektrum erfasst wurde, kann bei 124 ein Differenzspektrum berechnet werden. Falls mehr als zwei Messungen an dem biologischen Objekt vorgenommen wurden, kann eine entsprechend größere Anzahl von Messergebnissen verglichen werden. Beispielsweise können mehrere Raman-Spektren, die während oder nach Reizsetzung zeitsequentiell erfasst wurden, jeweils mit einem Raman-Spektrum, das vor Reizsetzung an demselben biologischen Objekt ermittelt wurde, verglichen werden.

Bei Schritt 125 wird das biologische Objekt anhand des Vergleichs von vor Reizsetzung und während oder nach Reizsetzung erhaltenen Messergebnissen charakterisiert. Dazu kann beispielsweise ein Differenzspektrum, das bei Schritt 124 ermittelt wurde, mit einem Referenz-Differenzspektrum verglichen werden, das in einer Datenbank abgelegt ist.

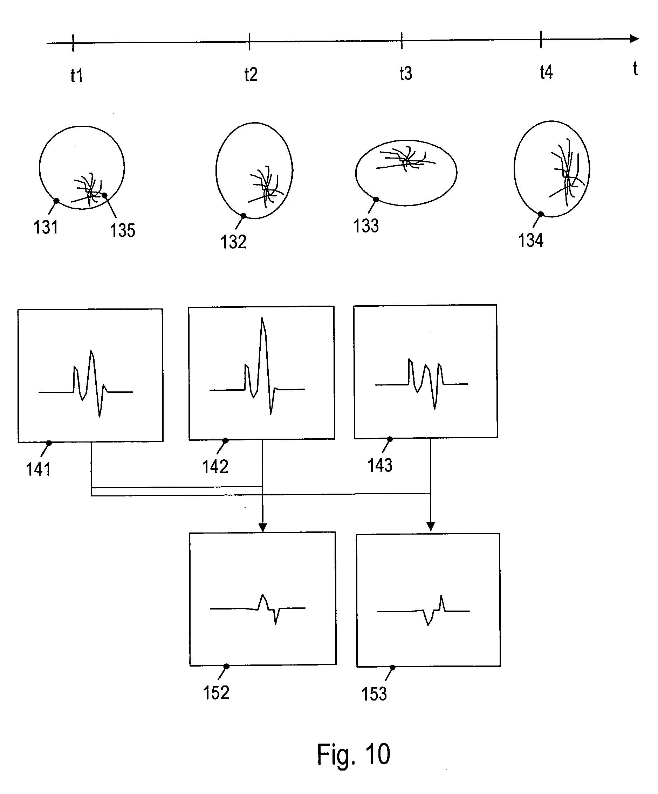

Fig. 10 illustriert die Durchführung des Verfahrens nach einem Ausführungsbeispiel. Dabei sind Zustände 131-134 eines biologischen Objekts zu verschiedenen Zeiten t1, t2, t3 und t4 dargestellt. Raman-Spektren 141-143 werden zu Zeiten t1, t2 und t3 erfasst. Aus den Raman-Spektren 141 und 142 kann ein Differenzspektrum 152 zwischen dem bei t2 erfassten Raman-Spektrum 142 und dem bei t1 erfassten Raman-Spektrum 141 ermittelt werden. Aus den Raman-Spektren 141 und 143 kann ein Differenzspektrum 153 zwischen dem bei t3 erfassten Raman-Spektrum 143 und dem bei t1 erfassten Raman-Spektrum 141 ermittelt werden.

Wie schematisch dargestellt, kann das biologische Objekt so stimuliert werden, dass es in Schwingungen versetzt wird. Bei der Zeit t1 weist das biologische Objekt, beispielsweise eine Zelle, einen Zustand 131 auf. Mit einer bei der Zeit t1 durchgeführten Raman-Spektroskopie können Informationen über intrinsische Eigenschaften des biologischen Objekts, beispielsweise über eine Dichte, Menge oder Art bestimmter Moleküle 135 in einer Zelle, erhalten werden.

Um das biologische Objekt zu Schwingungen anzuregen, kann eine pulsierende Kraft auf das biologische Objekt ausgeübt werden. Durch die Anregung zu einer Schwingung, insbesondere durch Anregung einer Resonanzschwingung, wird ein Reiz auf das biologische Objekt ausgeübt. Dazu kann beispielsweise ein Anregungsstrahl des Raman-Spektrometers gepulst mit einer Repetitionsrate auf das biologische Objekt eingestrahlt werden. Die Repetitionsrate kann variiert werden, bis eine Resonanz erreicht ist.

Zustände des biologischen Objekts zu verschiedenen Zeiten t2, t3 und t4 des Schwingungszyklus sind bei 132-134 dargestellt. Beispielhaft ist eine Deformationsschwingung dargestellt. Durch die Schwingung wird eine Veränderung im Inneren des biologischen Objekts hervorgerufen, die zusätzlich zu einer Volumen- und/oder Formänderung des biologischen Objekts auftreten kann. Beispielsweise kann sich eine Dichte und/oder Lage der Moleküle 135 in einem Zellinneren als Funktion der Zeit ändern. Es kann sich auch eine Art der Moleküle 135 im Zellinneren ändern. Derartige Änderungen führen zu einer Verschiebung von Spektrallinien und/oder einer Veränderung spektraler Gewichte der während der Schwingung bei t2 und t3 erfassten Raman-Spektren 142, 143 im Vergleich zu dem bei t1 erfassten Raman-Spektrum 141. Alternativ oder zusätzlich können bei der Raman-Spektroskopie, die zu unterschiedlichen Zeiten durchgeführt wird, Anregungsstrahlen mit unterschiedlicher Polarisation verwendet werden.

Während Ausführungsbeispiele unter Bezugnahme auf die Figuren beschrieben wurden, können Abwandlungen bei weiteren Ausführungsbeispielen realisiert sein. Während Vorrichtungen detailliert beschrieben wurden, bei denen sowohl eine DHMI-Datenerfassung als auch eine Raman-Spektroskopie durchgeführt wird, um eine Reaktion eines biologischen Objekts auf einen Reiz zu bestimmen, kann bei weiteren Ausführungsformen auf die Durchführung der DHMI-Datenerfassung verzichtet werden. Beispielsweise kann es bei weiteren Anwendungen ausreichend sein, das biologische Objekt im Hinblick auf die Änderung seines Raman-Spektrums als Reaktion auf den Reiz zu charakterisieren. In diesem Fall kann eine Raman-Spektroskopie alle zur Charakterisierung erforderlichen Daten liefern.

Während Ausführungsbeispiele beschrieben wurden, bei denen die Charakterisierung biologischer Objekte nach einem Datenbankabgleich zu einer weiteren Maßnahme, beispielsweise zu einer Sortierung der biologischen Objekte eingesetzt wird, kann bei weiteren Ausführungsformen die Charakterisierung mit den hier beschriebenen Verfahren und Vorrichtungen auch eingesetzt werden, um eine Datenbasis für die automatische Sortierung zu generieren oder um Kenntnisse über den Aufbau von biologischen Objekten zu erhalten.

Während Ausführungsbeispiele beschrieben wurden, bei denen verschiedene optische Signale, beispielsweise der Anregungsstrahl für die Raman-Spektroskopie, über verschiedene Positionen gerastert wird, kann bei weiteren Ausführungsformen auf eine Rasterung verzichtet werden.

Während Ausführungsbeispiele beschrieben wurden, bei denen ein Reiz unter Verwendung optischer Strahlung erzeugt wurde, kann bei weiteren Ausführungsformen der Reiz auch anderweitig erzeugt werden, beispielsweise durch Ultraschall, Hochfrequenzpuls, elektromagnetische Strahlung oder die Gabe von Wirkstoffen bzw. Medikamenten. Der Reiz kann auch durch das Zusammenspiel von optischer Strahlung und Fluidströmung erzeugt werden. Beispielsweise können von einem Fluidstrom auf ein in einer optischen Pinzette gefangenes Objekt ausgeübte Scherkräfte eine Verformung des Objekts hervorrufen. Es kann eine Raman-Spektroskopie und vorzugsweise zusätzlich eine Datenerfassung mit DHMI als Reaktion auf verschiedene Reize durchgeführt werden. Es kann zusätzlich oder alternativ auch die Reaktion auf die Einstrahlung von Ultraschall oder auf ein elektrisches oder magnetisches Hochfrequenzfeld ermittelt werden.

## Patentansprüche

1. Verfahren zum Charakterisieren eines biologischen Objekts (8, 9; 8, 90), wobei
berührungslos ein Reiz auf das biologische Objekt (8, 9; 8, 90) ausgeübt wird, mehrere Messungen an dem biologischen Objekt (8, 9; 8, 90) durchgeführt werden, um eine Reaktion des biologischen Objekts (8, 9; 8, 90) auf den Reiz zu ermitteln, wobei zum Ermitteln der Reaktion
- eine Messung, die eine Erfassung einer Raman-Streuung umfasst, vor Ausüben des Reizes an dem biologischen Objekt (8, 9; 8, 90) durchgeführt wird und
- eine weitere Messung, die eine weitere Erfassung der Raman-Streuung umfasst, nach oder während Ausüben des Reizes an dem biologischen Objekt (8, 9; 8, 90) durchgeführt wird,
wobei ein Vergleich von Resultaten der Messung und der weiteren Messung durchgeführt wird,
**dadurch gekennzeichnet,**
**dass** das biologische Objekt (8, 9; 8, 90) abhängig von dem Vergleich der Resultate der Messungen charakterisiert wird.

2. Verfahren nach Anspruch 1,
wobei die Messung und die weitere Messung sowohl die Erfassung einer Raman-Streuung als auch eine Datenerfassung mittels digitaler holographischer Mikrointerferometrie, DHMI, umfasst.

3. Verfahren nach Anspruch 1 oder 2,
wobei in der Messung ein Raman-Spektrum (141) und in der weiteren Messung ein weiteres Raman-Spektrum (142, 143) erfasst wird,
wobei ein Differenzspektrum (152, 153) zwischen dem Raman-Spektrum (141) und dem weiteren Raman-Spektrum (142, 143) ermittelt wird, und
wobei das biologische Objekt (8, 9; 8, 90) anhand des Differenzspektrums (152, 153) charakterisiert wird.

4. Verfahren nach einem der vorhergehenden Ansprüche,
wobei ein Hintergrund-Raman-Spektrum eines Substrats, an dem das biologische Objekt (8, 9; 8, 90) angeordnet ist, erfasst wird, und
wobei das Hintergrund-Raman-Spektrum von einem in der Messung erfassten Raman-Spektrum und von einem in der weiteren Messung erfassten weiteren Raman-Spektrum subtrahiert wird.

5. Verfahren nach einem der vorhergehenden Ansprüche,
wobei zum Ausüben des Reizes elektromagnetische Strahlung (79, 80; 104-107) auf das biologische Objekt (8, 9; 8, 90) eingestrahlt wird.

6. Verfahren nach einem der vorhergehenden Ansprüche,
wobei zum Ausüben des Reizes ein Laserstrahl oder mehrere Laserstrahlen (79, 80; 104-107; 115) auf das biologische Objekt (8, 9; 8, 90) eingestrahlt wird bzw. werden, wobei der Laserstrahl oder die mehreren Laserstrahlen (79, 80; 104-107; 115) gepulst auf das biologische Objekt (8, 9; 8, 90) eingestrahlt wird bzw. werden, wobei eine Repetitionsrate des Laserstrahls oder der mehreren Laserstrahlen (79, 80; 104-107; 115) variiert wird, um eine Resonanzschwingung des biologischen Objekts anzuregen.

7. Verfahren nach einem der vorhergehenden Ansprüche,
wobei zum Ausüben des Reizes ein Laserstrahl auf das biologische Objekt (8, 9; 8, 90) eingestrahlt wird, wobei der Laserstrahl (79, 80; 104-107) über mehrere verschiedene Kanäle (82, 86) eines Mikrofluidiksystems (81) gerastert wird, um den Reiz auf mehrere biologische Objekte (8, 90) auszuüben,
wobei unter Verwendung des Laserstrahls (79, 80; 104-107; 115) eine Verformung des biologischen Objekts (8, 9; 8, 90) hervorgerufen wird, wobei eine Leistungsdichte des Laserstrahls (79, 80; 104-107; 115) an dem biologischen Objekt (8, 9; 8, 90) so gewählt wird, dass das biologische Objekt (8, 9; 8, 90) nicht zerstört wird.

8. Verfahren nach einem der vorhergehenden Ansprüche,
wobei zum Ausüben des Reizes ein Laserstrahl auf das biologische Objekt (8, 9; 8, 90) eingestrahlt wird, wobei der Laserstrahl das biologische Objekt (8, 9; 8, 90) in einem Fluidstrom an einer Position hält, und wobei der Laserstrahl und eine Strömungsgeschwindigkeit des Fluidstroms so eingestellt werden, dass der Fluidstrom eine Verformung des biologischen Objekts (8, 9; 8, 90) hervorruft.

9. Verfahren nach einem der vorhergehenden Ansprüche,
wobei der Reiz mittels Ultraschallwellen oder mittels eines elektrischen oder magnetischen Hochfrequenzfeldes ausgeübt wird.

10. Verfahren nach einem der vorhergehenden Ansprüche,
wobei der Reiz chemisch, insbesondere durch Gabe von Wirkstoffen oder Medikamenten, ausgelöst wird.

11. Verfahren nach einem der vorhergehenden Ansprüche,
wobei mittels DHMI weiterhin ein Volumen und/oder eine Form des biologischen Objekts (8, 9; 8, 90) als Reaktion auf den Reiz bestimmt wird, und
wobei durch die Erfassung der Raman-Streuung an dem biologischen Objekt (8, 9; 8, 90) jeweils eine Molekülansammlung und/oder eine Molekülzusammensetzung, insbesondere eine Proteinzusammensetzung, in einem Kern und/oder Zytoplasma einer Zelle bestimmt wird.

12. Verfahren nach einem der vorhergehenden Ansprüche,
wobei die Messung und die weitere Messung an dem biologische Objekt (8, 9; 8, 90) durchgeführt werden, während das biologische Objekt (8, 9; 8, 90) in einem Fluidkanal (13; 82, 86) eines Mikrofluidiksystems (11; 81) positioniert ist,
wobei das Mikrofluidiksystem (11; 81) einen geschlossenen Fluidkreislauf (12) umfasst, in dem biologische Objekte vor Durchführung der Messung transportiert, insbesondere kontinuierlich transportiert, werden.

13. Vorrichtung zum Charakterisieren eines biologischen Objekts (8, 9; 8, 90), umfassend
eine Einrichtung (40; 73-76; 93-96) zum berührungslosen Erzeugen eines Reizes auf das biologische Objekt (8, 9; 8, 90), und
eine Messeinrichtung (22, 24a, 24b, 24c; 22, 24a, 24b, 38) zum Durchführen einer Messung an dem biologischen Objekt (8, 9; 8, 90), um eine Reaktion des biologischen Objekts (8, 9; 8, 90) auf den Reiz zu ermitteln, wobei die Messeinrichtung (22, 24a, 24b, 24c; 22, 24a, 24b, 38) für eine Erfassung einer Raman-Streuung an dem biologischen Objekt (8, 9; 8, 90) eingerichtet ist,
wobei die Messeinrichtung eingerichtet ist, um zum Ermitteln der Reaktion des biologischen Objekts (8, 9; 8, 90) auf den Reiz
- eine Erfassung der Raman-Streuung vor Ausüben des Reizes durchzuführen und
- eine weitere Erfassung der Raman-Streuung nach oder während Ausüben des Reizes durchzuführen,
**gekennzeichnet durch** eine Auswertelogik (25), die eingerichtet ist, um Resultate der vor Ausüben des Reizes durchgeführten Messung und der nach oder während Ausüben des Reizes durchgeführten weiteren Messung zu vergleichen, und um das biologische Objekt (8, 9; 8, 90) abhängig von dem Vergleich der Resultate der Messungen zu charakterisieren.

14. Vorrichtung nach Anspruch 13,
wobei die Messeinrichtung eine Raman-Einrichtung (22) zum Erfassen einer Raman-Streuung an dem biologischen Objekt (8, 9; 8, 90) und eine digitale holographische Mikrointerferometrie, DHMI,-Einrichtung (24a, 24b, 24c; 24a, 24b, 38) zum Erfassen einer DHMI-Aufnahme des biologischen Objekts (8, 9; 8, 90) umfasst,
wobei die Vorrichtung ein optisches Mikroskop umfasst, und
wobei die Raman-Einrichtung (22) eingerichtet ist, um einen Fokus eines Anregungsstrahls (55) der Raman-Einrichtung unabhängig von einem Fokus eines Strahlengangs des optischen Mikroskops in drei orthogonalen Raumrichtungen steuerbar zu bewegen.

15. Vorrichtung nach Anspruch 13 oder Anspruch 14,
welche zur Durchführung des Verfahrens nach einem der Ansprüche 1-12 eingerichtet ist.

## Claims

1. A method for characterizing a biological object (8, 9; 8, 90), wherein
a stimulus is applied to the biological object (8, 9; 8, 90) in a contactless fashion, plural measurements are carried out on the biological object (8, 9; 8, 90) to ascertain a response of the biological object (8, 9; 8, 90) to the stimulus, wherein, to ascertain the response,
- a measurement which comprises a detection of Raman scattering is performed on the biological object (8, 9; 8, 90) prior to application of the stimulus, and
- a further measurement which comprises a further detection of Raman scattering is performed on the biological object (8, 9; 8, 90) after or during application of the stimulus,
wherein a comparison of results of the measurement and the further measurement is performed,
**characterized in that**
the biological object (8, 9; 8, 90) is characterized as a function of the comparison of the results of the measurements.

2. The method according to claim 1,
wherein the measurement and the further measurement comprise both the detection of a Raman scattering and data acquisition by means of digital holographic microinterferometry, DHMI.

3. The method according to claim 1 or 2,
wherein a Raman spectrum (141) is captured in the measurement and a further Raman spectrum (142, 143) is captured in the further measurement,
wherein a difference spectrum (152, 153) of the Raman spectrum (141) and the further Raman spectrum (142, 143) is determined, and
wherein the biological object (8, 9; 8, 90) is characterized based on the difference spectrum (152, 153).

4. The method according to any one of the preceding claims,
wherein a background Raman spectrum of a substrate on which the biological object (8, 9; 8, 90) is arranged is detected, and
wherein the background Raman spectrum is subtracted from a Raman spectrum detected in the measurement and from a further Raman spectrum detected in the further measurement.

5. The method according to any one of the preceding claims,
wherein electromagnetic radiation (79, 80; 104-107) is irradiated onto the biological object (8, 9; 8, 90) to apply the stimulus.

6. The method according to any one of the preceding claims,
wherein a laser beam or plural laser beams (79, 80; 104-107; 115) are irradiated onto the biological object (8, 9; 8, 90) to apply the stimulus,
wherein the laser beam or the plural laser beams (79, 80; 104-107; 115) are irradiated onto the biological object (8, 9; 8, 90) in a pulsed fashion,
wherein a repetition rate of the laser beam or of the plural laser beams (79, 80; 104-107; 115) is varied to excite a resonance vibration of the biological object.

7. The method according to any one of the preceding claims,
wherein a laser beam is irradiated onto the biological object (8, 9; 8, 90) to apply the stimulus, wherein the laser beam (79, 80; 104-107) is scanned over plural different channels (82, 86) of a microfluidic system (81) to apply the stimulus to plural biological obejcts (8, 90),
wherein a deformation of the biological object (8, 9; 8, 90) is induced using the laser beam (79, 80; 104-107; 115), wherein a power density of the laser beam (79, 80; 104-107; 115) at the biological object (8, 9; 8, 90) is selected such that the biological object (8, 9; 8, 90) is not destroyed.

8. The method according to any one of the preceding claims,
wherein a laser beam is irradiated onto the biological object (8, 9; 8, 90) to apply the stimulus, wherein the laser beam holds the biological object (8, 9; 8, 90) at a position in a fluid flow, and wherein the laser beam and a flow speed of the fluid flow are set such that the fluid flow induces a deformation of the biological object (8, 9; 8, 90).

9. The method according to any one of the preceding claims,
wherein the stimulus is applied using ultrasonic waves or using an electric or magnetic high frequency field.

10. The method according to any one of the preceding claims,
wherein the stimulus is triggered chemically, in particular by administration of agents or drugs.

11. The method according to any one of the preceding claims,
wherein a volume and/or a shape of the biological object (8, 9; 8, 90) in response to the stimulus is determined by means of DHMI, and
wherein, by detecting the Raman scattering on the biological object (8, 9; 8, 90), an agglomeration of molecules and/or a molecular composition, in particular a protein composition, in a nucleus and/or a cytoplasm of a cell is respectively determined.

12. The method according to any one of the preceding claims,
wherein the measurement and the further measurement are performed on the biological object (8, 9; 8, 90) while the biological object (8, 9; 8, 90) is positioned in a fluid channel (13; 82, 86) of a microfluidic system (11; 81),
wherein the microfluidic system (11; 81) comprises a closed fluid loop (12) in which biological objects are transported, in particular continuously transported, before the measurement is performed.

13. An apparatus for characterizing a biological object (8, 9; 8, 90), comprising
a device (40; 73-76; 93-96) for generating a stimulus on the biological object (8, 9; 8, 90) in a contactless fashion, and
a measurement device (22, 24a, 24b, 24c; 22, 24a, 24b, 38) for performing a measurement on the biological object (8, 9; 8, 90) to ascertain a response of the biological object (8, 9; 8, 90) to the stimulus, wherein the measurement device (22, 24a, 24b, 24c; 22, 24a, 24b, 38) is configured for data acquisition by means of Raman scattering on the biological object (8, 9; 8, 90),
wherein the measurement device is configured, for ascertaining the response of the biological object (8, 9; 8, 90) to the stimulus,
- to perform a detection of the Raman scattering before application of the stimulus, and
- to perform a further detection of the Raman scattering after or during application of the stimulus,
**characterized by**
an evaluation logic (25) which is configured to compare results of the measurement performed prior to application of the stimulus and of the further measurement performed after or during application of the stimulus, and to characterize the biological object (8, 9; 8, 90) as a function of the comparison of the results of the measurements.

14. The apparatus according to claim 13,
wherein the measurement device comprises a Raman device (22) for detecting a Raman scattering on the biological object (8, 9; 8, 90) and a digital holographic microinterferometry, DHMI, device (24a, 24b, 24c; 24a, 24b, 38) for capturing a DHMI image of the biological object (8, 9; 8, 90),
wherein the apparatus comprises an optical microscope, and
wherein the Raman device (22) is configured to controllably move a focus of an excitation beam (55) of the Raman device in three orthogonal spatial directions independently of a focus of a beam path of the optical microscope.

15. The apparatus according to claim 13 or claim 14,
configured to perform the method according to any one of claims 1-12.

## Revendications

1. Procédé servant à caractériser un objet biologique (8, 9 ; 8, 90), sachant que
un stimulus est appliqué sur l'objet biologique (8, 9 ; 8, 90) sans contact,
plusieurs mesures sont effectuées sur l'objet biologique (8, 9 ; 8, 90) afin de déterminer une réaction de l'objet biologique (8, 9 ; 8, 90) au stimulus, sachant que pour déterminer la réaction
- une mesure incluant une détection d'une diffusion Raman est effectuée avant d'appliquer le stimulus sur l'objet biologique (8, 9 ; 8, 90) et
- une mesure supplémentaire incluant une détection supplémentaire de la diffusion Raman est effectuée après ou au cours de l'application du stimulus sur l'objet biologique (8, 9 ; 8, 90),
sachant qu'une comparaison entre les résultats de la mesure et la mesure supplémentaire est effectuée,
**caractérisé en ce**
**que** l'objet biologique (8, 9 ; 8, 90) est caractérisé en fonction de la comparaison des résultats des mesures.

2. Procédé selon la revendication 1,
sachant que la mesure et la mesure supplémentaire incluent aussi bien la détection d'une diffusion Raman qu'une détection de données au moyen d'une microinterférométrie holographique numérique (DHMI).

3. Procédé selon la revendication 1 ou 2,
sachant qu'un spectre Raman (141) est détecté lors de la mesure et qu'un spectre Raman supplémentaire (142, 143) est détecté lors de la mesure supplémentaire,
sachant qu'un spectre différentiel (152, 153) est déterminé entre le spectre Raman (141) et le spectre Raman supplémentaire (142, 143), et
sachant que l'objet biologique (8, 9 ; 8, 90) est caractérisé à l'aide du spectre différentiel (152, 153).

4. Procédé selon l'une quelconque des revendications précédentes,
sachant qu'un spectre de Raman contextuel d'un substrat, sur duquel est disposé l'objet biologique (8, 9 ; 8, 90), est détecté, et
sachant que le spectre Raman contextuel est soustrait d'un spectre Raman détecté lors de la mesure et d'un spectre Raman supplémentaire détecté lors de la mesure supplémentaire.

5. Procédé selon l'une quelconque des revendications précédentes,
sachant qu'un rayonnement électromagnétique (79, 80 ; 104-107) est irradié sur l'objet biologique (8, 9 ; 8, 90) afin d'appliquer le stimulus.

6. Procédé selon l'une quelconque des revendications précédentes,
sachant qu'un rayon laser ou plusieurs rayons laser (79, 80 ; 104-107 ; 115) sont irradiés sur l'objet biologique (8, 9 ; 8, 90) afin d'appliquer le stimulus, sachant que le rayon laser ou les nombreux rayons laser (79, 80 ; 104-107 ; 115) sont irradiés de manière pulsée sur l'objet biologique (8, 9 ; 8, 90), sachant qu'un indice de répétition du rayon laser ou des nombreux rayons laser (79, 80 ; 104-107 ; 115) varie afin de stimuler une oscillation de résonance de l'objet biologique.

7. Procédé selon l'une quelconque des revendications précédentes,
sachant qu'un rayon laser est irradié sur l'objet biologique (8, 9 ; 8, 90) afin d'appliquer le stimulus, sachant que le rayon laser (79, 80 ; 104 - 107) est quadrillé par l'intermédiaire de plusieurs canaux (82, 86) différents d'un système microfluidique (81), afin d'appliquer le stimulus sur plusieurs objets biologiques (8, 90),
sachant qu'une déformation de l'objet biologique (8, 9 ; 8, 90) est provoquée en utilisant le rayon laser (79, 80 ; 104 -17 ; 115), sachant qu'une densité de puissance du rayon laser (79, 80 ; 104 - 107 ; 115) sur l'objet biologique (8, 9 ; 8, 90) est choisie de manière à ne pas détruire l'objet biologique (8, 9 ; 8, 90).

8. Procédé selon l'une quelconque des revendications précédentes,
sachant qu'un rayon laser est irradié sur l'objet biologique (8, 9 ; 8, 90) afin d'appliquer le stimulus, sachant que le rayon laser retient l'objet biologique (8, 9 ; 8, 90) dans un flux de fluide au niveau d'une position, et sachant que le rayon laser et une vitesse d'écoulement du flux de fluide sont ajustés de telle manière que le flux de fluide provoque une déformation de l'objet biologique (8, 9 ; 8, 90).

9. Procédé selon l'une quelconque des revendications précédentes,
sachant que le stimulus est appliqué au moyen d'ondes à ultrasons ou au moyen d'un champ haute fréquence électrique ou magnétique.

10. Procédé selon l'une quelconque des revendications précédentes,
sachant que le stimulus est déclenché de manière chimique, en particulier par l'ajout de principes actifs ou de médicaments.

11. Procédé selon l'une quelconque des revendications précédentes,
sachant qu'en outre un volume et/ou une forme de l'objet biologique (8, 9 ; 8, 90) sont déterminés en tant que réaction au stimulus au moyen de la DHMI, et
sachant que respectivement une accumulation de molécules et/ou une composition de molécules, en particulier une composition de protéines, sont déterminées dans un noyau et/ou un cytoplasme d'une cellule par la détection de la diffusion Raman au niveau de l'objet biologique (8, 9 ; 8, 90).

12. Procédé selon l'une quelconque des revendications précédentes,
sachant que la mesure et la mesure supplémentaire sont effectuées sur l'objet biologique (8, .9 ; 8, 90), tandis que l'objet biologique (8, 9 ; 8, 90) est positionné dans un canal de fluide (13 ; 82, 86) d'un système microfluidique (11 ; 81),
sachant que le système microfluidique (11 ; 81) comprend un circuit fluidique (12) fermé, dans lequel des objets biologiques sont transportés, en particulier sont transportés en continu avant que la mesure ne soit effectuée.

13. Dispositif servant à caractériser un objet biologique (8, 9 ; 8, 90) comprenant
un système (40 ; 73 - 76 ; 93- 96) servant à produire sans contact un stimulus sur l'objet biologique (8, 9 ; 8, 90), et
un système de mesure (22, 24a, 24b, 24c ; 22, 24a, 24b, 38) servant à effectuer une mesure sur l'objet biologique (8, 9 ; 8, 90) afin de déterminer une réaction de l'objet biologique (8, 9 ; 8, 90) au stimulus, sachant que le système de mesure (22, 24a, 24b, 24c ; 22, 24a, 24b, 38) est mis au point en vue d'une détection d'une diffusion Raman sur l'objet biologique (8, 9 ; 8, 90),
sachant que le système de mesure est mis au point afin
- d'effectuer une détection de la diffusion Raman avant d'appliquer le stimulus,
- d'effectuer une détection supplémentaire de la diffusion Raman avant ou au cours de l'application du stimulus,
afin de déterminer la réaction de l'objet biologique (8, 9 ; 8, 90) au stimulus,
**caractérisé par** une logique d'analyse (25) qui est mise au point pour comparer des résultats de la mesure effectuée avant l'application du stimulus et des résultats de la mesure supplémentaire effectuée après ou au cours de l'application du stimulus et pour caractériser l'objet biologique (8, 9 ; 8, 90) en fonction de la comparaison des résultats des mesures.

14. Dispositif selon la revendication 13,
sachant que le système de mesure comprend un système Raman (22) servant à détecter une diffusion Raman au niveau de l'objet biologique (8, 9 ; 8, 90) et un système de microinterférométrie holographique numérique (DHMI) (24a, 24b, 24c ; 24a, 24b, 38) servant à détecter une prise de vue par DHMI de l'objet biologique (8, 9 ; 8, 90),
sachant que le dispositif comprend un microscope optique, et
sachant que le système Raman (22) est mis au point afin de déplacer de manière commandable un foyer d'un rayon de stimulation (55) du système Raman en fonction d'un foyer d'une trajectoire de rayons du microscope optique dans trois directions spatiales orthogonales.

15. Dispositif selon la revendication 13 ou la revendication 14,
qui est mis au point aux fins de la mise en oeuvre du procédé selon l'une quelconque des revendications 1 à 12.
